# EUROPEAN PATENT APPLICATION

(11) **EP 4 123 013 A1**
(43) Date of publication of application: **25.01.2023**
(21) Application number: 21771431.0
(22) Date of filing: 15.03.2021
(51) Int. Cl.: C12N 5/07, C12P 21/00

(54) **ANIMAL CELL CULTURING METHOD**

(30) Priority: 16.03.2020 JP 2020045363
(71) Applicant: AJINOMOTO CO., INC., Chuo-ku Tokyo 104-8315 (JP)
(72) Inventor: SATO, Seiichi, Kawasaki-shi, Kanagawa 210-8681 (JP); AKEDA, Yuki, Kawasaki-shi, Kanagawa 210-8681 (JP); ENDO, Yuta, Kawasaki-shi, Kanagawa 210-8681 (JP); MATSUBA, Ayana, Kawasaki-shi, Kanagawa 210-8681 (JP); TSUJI, Chihiro, Kawasaki-shi, Kanagawa 210-8681 (JP)
(74) Representative: Strehl Schübel-Hopf & Partner
(86) International application number: PCT/JP2021/010343
(87) International publication number: WO 2021/187411

(57) **Abstract**

A method for producing an objective protein by using animal cells as an expression host is provided. The objective protein is produced by culturing animal cells having an objective protein-producing ability in the presence of an L-cysteine derivative such as (2RS,4R)-2-methyl-2,4-thiazolidinedicarboxylic acid 2-ethyl ester.

## Description

### Technical Field

The present invention relates to a method for culturing animal cells. An embodiment of the present invention relates to a method for producing an objective substance, such as a protein, by animal cells.

### Background Art

Culture media containing amino acids such as cysteine (Cys) are widely used for culturing animal cells. Cys is spontaneously oxidized in a culture medium, to be converted to cystine ((Cys)2). Since (Cys)2 has low solubility, it tends to precipitate in a liquid culture medium. Therefore, Cys is a factor in the storage instability of liquid culture media.

Cys can condense with α-keto acids such as pyruvic acid (Pyr) and α-ketoglutaric acid to form thiazolidine derivatives (Non-Patent Document 1). It have been reported that use of α-keto acids stabilizes culture media, and that thiazolidine derivatives are effective for culturing animal cells (Non-Patent Document 1).

Cysteine derivatives such as (2RS,4R)-2-methyl-2,4-thiazolidinedicarboxylic acid 2-ethyl ester and (2RS,4R)-2-methyl-2,4-thiazolidinedicarboxylic acid are known as ingredients in cosmetics (Patent Document 1).

### Prior art references

### Patent documents

Patent document 1: JP patent No. 5439849

### Non-Patent documents

Non-Patent document 1: Kuschelewski J et al., Antioxidant effect of thiazolidine molecules in cell culture media improves stability and performance. Biotechnol Prog. 2017 May;33(3):759-770.

### Summary of Invention

### Object to be Achieved by the Invention

An object of the present invention is to provide a novel technique relating to culture of animal cells.

### [Means for Achieving the Object]

The inventors of the present invention found that specific cysteine derivatives function as alternatives to cysteine upon culturing animal cells and accomplished the present invention.

The present invention can be thus embodied as follows.
[1] A culture medium for culturing animal cells, the culture medium containing an active ingredient,
   wherein the active ingredient is a compound shown by the formula (I) described later:
   wherein, in the formula (I), "R₁" represents a C1-22 alkyl group that may have a substituent, and "R₂" represents a hydrogen atom, a C6-12 aryl group that may have a substituent, or a linear C1-6 alkyl group that may have a substituent.
[2] The culture medium mentioned above, wherein:
   "R₁" represents a C1-12 alkyl group, or a C1-6 alkyl group substituted with a C6-12 aryl group that may have a substituent, and/or
   "R₂" represents a hydrogen atom, a linear C1-6 alkyl group, a linear C1-6 alkyl group substituted with a C6-12 aryl group that may have a substituent, or a linear C1-6 alkyl group substituted with a C1-7 acyl group.
[3] The culture medium mentioned above, wherein the active ingredient is a compound selected from the group consisting of (2RS,4R)-2-methyl-2,4-thiazolidinedicarboxylic acid 2-ethyl ester, (2RS,4R)-2-methyl-2,4-thiazolidinedicarboxylic acid 2-methyl ester, (2RS,4R)-2-methyl-2,4-thiazolidinedicarboxylic acid 2-i-propyl ester, (2RS,4R)-2-methyl-2,4-thiazolidinedicarboxylic acid 2-n-butyl ester, (2RS,4R)-2-methyl-2,4-thiazolidinedicarboxylic acid 2-t-butyl ester, (2RS,4R)-2-methyl-2,4-thiazolidinedicarboxylic acid 2-n-octyl ester, (2RS,4R)-2,4-thiazolidinedicarboxylic acid 2-ethyl ester, and (2RS,4R)-2-(4-hydroxyphenyl)methyl-2,4-thiazolidinedicarboxylic acid 2-methyl ester, and the 2R and 2S compounds thereof.
[4] The culture medium mentioned above, wherein the active ingredient is a compound selected from the group consisting of (2RS,4R)-2-methyl-2,4-thiazolidinedicarboxylic acid 2-ethyl ester and the 2R and 2S compounds thereof.
[5] The culture medium mentioned above, wherein the active ingredient is a compound selected from the group consisting of (2RS,4R)-2-methyl-2,4-thiazolidinedicarboxylic acid 2-methyl ester and the 2R and 2S compounds thereof.
[6] The culture medium mentioned above, wherein the concentration of the active ingredient is 0.92 mM or more.
[7] The culture medium mentioned above, wherein the concentration of the active ingredient is 5 mM or more.
[8] The culture medium mentioned above, wherein the concentration of the active ingredient is 10 mM or more.
[9] The culture medium mentioned above, wherein the culture medium is a feed medium.
[10] The culture medium mentioned above, wherein the culture medium is a starting medium.
[11] A method for producing an objective substance, the method comprising:
   culturing animal cells having an objective substance-producing ability in a culture medium; and
   collecting the objective substance,
   wherein the culturing is carried out in the presence of an active ingredient, and
   wherein the active ingredient is a compound shown by the formula (I) described later:
      wherein, in the formula (I), "R₁" represents a C1-22 alkyl group that may have a substituent, and "R₂" represents a hydrogen atom, a C6-12 aryl group that may have a substituent, or a linear C1-6 alkyl group that may have a substituent.
[12] The method mentioned above, wherein the objective substance is a protein.
[13] A method for culturing animal cells, the method comprising:
   culturing the animal cells in a culture medium,
   wherein the culturing is carried out in the presence of an active ingredient, and
   wherein the active ingredient is a compound shown by the formula (I) described later:
      wherein, in the formula (I), "R₁" represents a C1-22 alkyl group that may have a substituent, and "R₂" represents a hydrogen atom, a C6-12 aryl group that may have a substituent, or a linear C1-6 alkyl group that may have a substituent.
[14] The method mentioned above, wherein:
   "R₁" represents a C1-12 alkyl group, or a C1-6 alkyl group substituted with a C6-12 aryl group that may have a substituent, and/or
   "R₂" represents a hydrogen atom, a linear C1-6 alkyl group, a linear C1-6 alkyl group substituted with a C6-12 aryl group that may have a substituent, or a linear C1-6 alkyl group substituted with a C1-7 acyl group.
[15] The method mentioned above, wherein the active ingredient is a compound selected from the group consisting of (2RS,4R)-2-methyl-2,4-thiazolidinedicarboxylic acid 2-ethyl ester, (2RS,4R)-2-methyl-2,4-thiazolidinedicarboxylic acid 2-methyl ester, (2RS,4R)-2-methyl-2,4-thiazolidinedicarboxylic acid 2-i-propyl ester, (2RS,4R)-2-methyl-2,4-thiazolidinedicarboxylic acid 2-n-butyl ester, (2RS,4R)-2-methyl-2,4-thiazolidinedicarboxylic acid 2-t-butyl ester, (2RS,4R)-2-methyl-2,4-thiazolidinedicarboxylic acid 2-n-octyl ester, (2RS,4R)-2,4-thiazolidinedicarboxylic acid 2-ethyl ester, and (2RS,4R)-2-(4-hydroxyphenyl)methyl-2,4-thiazolidinedicarboxylic acid 2-methyl ester, and the 2R and 2S compounds thereof.
[16] The method mentioned above, wherein the active ingredient is a compound selected from the group consisting of (2RS,4R)-2-methyl-2,4-thiazolidinedicarboxylic acid 2-ethyl ester and the 2R and 2S compounds thereof.
[17] The method mentioned above, wherein the active ingredient is a compound selected from the group consisting of (2RS,4R)-2-methyl-2,4-thiazolidinedicarboxylic acid 2-methyl ester and the 2R and 2S compounds thereof.
[18] The method mentioned above, wherein the active ingredient is supplied to the culture medium after the start of the culturing.
[19] The method mentioned above, wherein the active ingredient is contained in the culture medium at the start of the culturing.
[20] The method mentioned above, wherein the concentration of the active ingredient in the culture medium during the culturing is 0.92 mM or more.
[21] The method mentioned above, wherein the concentration of the active ingredient in the culture medium during the culturing is 1.5 mM or more.
[22] The method mentioned above, wherein the active ingredient is supplied to the culture medium after the start of the culturing so as to be contained in the culture medium at said concentration.
[23] The method mentioned above, wherein the active ingredient is contained in the culture medium at said concentration at the start of the culturing.
[24] The method mentioned above,
   wherein the active ingredient is contained in the culture medium at said concentration in terms of an average value over a specific period of the culturing, and
   wherein the specific period is the whole period of the culturing or a period where the viable cell density of the animal cells in the culture medium is 5×10⁶ cells/mL or more.
[25] The method mentioned above,
   wherein the active ingredient is supplied to the culture medium at a supply amount of 0.1 mM or more per day over a specific period of the culturing, and
   wherein the specific period is the whole period of the culturing or a period where the viable cell density of the animal cells in the culture medium is 5×10⁶ cells/mL or more.
[26] The method mentioned above,
   wherein the active ingredient is supplied to the culture medium at a supply amount of 0.3 mM or more per day over a specific period of the culturing, and
   wherein the specific period is the whole period of the culturing or a period where the viable cell density of the animal cells in the culture medium is 5×10⁶ cells/mL or more.
[27] The method mentioned above, wherein the culturing comprises carrying out perfusion culture using a feed medium containing the active ingredient.
[28] The method mentioned above,
   wherein the culturing is carried out in the presence of a cysteine-related substance, and
   wherein the cysteine-related substance is selected from cysteine and derivatives thereof other than the active ingredient.
[29] The method mentioned above, wherein the cysteine-related substance is selected from cysteine, cystine, and cysteinyl pyruvic acid.
[30] The method mentioned above, wherein the cysteine-related substance is contained in the culture medium at the start of the culturing.
[31] A method for producing a compound shown by the formula (I) described later, the method comprising:
   reacting L-cysteine and a pyruvate derivative shown by the following formula (II) in a hydrous medium to thereby form crystals of the compound; and
   collecting the crystals:
      wherein, in the formulas (I) and (II), "R₁" represents a C1-C6 alkyl group, and "R₂" represents methyl group.

### Brief Description of Drawings

[FIG. 1] A diagram showing an effect of cysteine or a cysteine derivative in a basal medium on proliferation of CHO cells.
[FIG. 2] A diagram showing an effect of the concentration of cysteine or a cysteine derivative in a feed medium on proliferation of CHO cells.
[FIG. 3] A diagram showing an effect of the concentration of cysteine or a cysteine derivative in a feed medium on antibody production by CHO cells.
[FIG. 4] A diagram showing an effect of the concentration of cysteine or a cysteine derivative in a feed medium on proliferation of CHO cells.
[FIG. 5] A diagram showing an effect of the concentration of cysteine or a cysteine derivative in a feed medium on antibody production by CHO cells.
[FIG. 6] A diagram showing an effect of the concentration of cysteine or a cysteine derivative in a feed medium on proliferation of CHO cells.
[FIG. 7] A diagram showing an effect of the concentration of cysteine or a cysteine derivative in a feed medium on antibody production by CHO cells.

### Modes for Carrying out the Invention

### <1> Active ingredient

In the present invention, an active ingredient is used.

The active ingredient is a compound shown by the following formula (I):

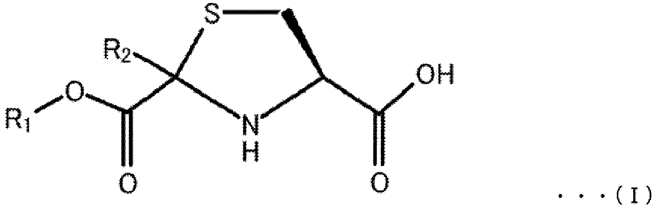

In the formula (I), "R₁" represents a C1-22 alkyl group that may have a substituent. In the formula (I), "R₂" represents a hydrogen atom, a C6-12 aryl group that may have a substituent, or a C1-6 alkyl group that may have a substituent. In the formula (I), "R₂" may represent, in particular, a hydrogen atom, a C6-12 aryl group that may have a substituent, or a linear C1-6 alkyl group that may have a substituent. The terms "R₁" and "R₂" may also be referred to as "R¹" and "R²", respectively.

That is, the C1-22 alkyl group of "R₁" may or may not have a substituent. In addition, the C6-12 aryl group or C1-6 alkyl group of "R₂" may or may not have a substituent.

Hereinafter, the term "C1-22 alkyl group" refers to a C1-22 alkyl group having no substituent unless otherwise stated, and the description concerning the C1-22 alkyl group having no substituent can also be applied similarly to a C1-22 alkyl group having a substituent. In addition, hereinafter, the term "C6-12 aryl group" refers to a C6-12 aryl group having no substituent unless otherwise stated, and the description concerning the C6-12 aryl group having no substituent can also be applied similarly to a C6-12 aryl group having a substituent. In addition, hereinafter, the term "C1-6 alkyl group" refers to a C1-6 alkyl group having no substituent unless otherwise stated, and the description concerning the C1-6 alkyl group having no substituent can also be applied similarly to a C1-6 alkyl group having a substituent. The same shall apply to other functional groups that may have a substituent.

The phrase "a C1-22 alkyl group has a substituent" means that one or more of hydrogen atoms constituting the C1-22 alkyl group has been substituted with a substituent. The phrase "a C6-12 aryl group has a substituent" means that one or more of hydrogen atoms constituting the C6-12 aryl group has been substituted with a substituent. The phrase "a C1-6 alkyl group has a substituent" means that one or more of hydrogen atoms constituting the C1-6 alkyl group has been substituted with a substituent. The number of hydrogen atoms to be substituted with a substituent in the C1-22 alkyl group of "R₁", the C6-12 aryl group of "R₂", or C1-6 alkyl group of "R₂" may be, for example, 1 to 5, 1 to 4, 1 to 3, or 1 to 2, and may specifically be 1, 2, 3, 4, or 5. The number of hydrogen atoms to be substituted with a substituent in the C1-22 alkyl group of "R₁", the C6-12 aryl group of "R₂", or C1-6 alkyl group of "R₂" may also be read as the number of substituents comprised in the C1-22 alkyl group of "R₁", the C6-12 aryl group of "R₂", or C1-6 alkyl group of "R₂". When two or more hydrogen atoms are substituted with a substituent, the substituent is independently selected for each hydrogen atom. The same shall apply to other functional groups that may have a substituent.

The term "C1-22 alkyl group" refers to an alkyl group having a carbon number of 1 to 22. The C1-22 alkyl group may be linear or branched. The carbon number of the C1-22 alkyl group may be 1, 2, 3, 4, 5, 6, 7, 8, 9, 10, 11, 12, 13, 14, 15, 16, 17, 18, 19, 20, 21, or 22. The carbon number of the C1-22 alkyl group, for example, may be 1 or more, 2 or more, 3 or more, 4 or more, 5 or more, 6 or more, 7 or more, 8 or more, 9 or more, or 10 or more, may be 22 or less, 21 or less, 20 or less, 19 or less, 18 or less, 17 or less, 16 or less, 15 or less, 14 or less, 13 or less, 12 or less, 11 or less, 10 or less, 9 or less, 8 or less, 7 or less, 6 or less, 5 or less, or 4 or less, or may be within a range defined as a non-contradictory combination thereof. The carbon number of the C1-22 alkyl group may be, specifically, for example, 1 to 22, 1 to 12, or 1 to 6. An alkyl group having a carbon number of n is also referred to as "Cn alkyl group". For example, the term "C1-12 alkyl group" refers to an alkyl group having a carbon number of 1 to 12. Also, the term "C1-6 alkyl group" refers to an alkyl group having a carbon number of 1 to 6. Examples of the C1-22 alkyl group include methyl group, ethyl group, isopropyl group, propyl group, butyl group, isobutyl group, sec-butyl group, tert-butyl group, pentyl group, sec-pentyl group, tert-pentyl group, isopentyl group, hexyl group, heptyl group, octyl group, 2-ethylhexyl group, tert-octyl group, nonyl group, isononyl group, decyl group, isodecyl group, undecyl group, dodecyl group, tridecyl group, isotridecyl group, tetradecyl group, pentadecyl group, hexadecyl group, isohexadecyl group, heptadecyl group, octadecyl group, isooctadecyl group, oleyl group, and behenyl group. Particular examples of the C1-22 alkyl group include methyl group, ethyl group, and isopropyl group.

The term "C1-6 alkyl group" refers to an alkyl group having a carbon number of 1 to 6. The C1-6 alkyl group may be linear or branched. The C1-6 alkyl group may be, in particular, linear. Examples of the C1-6 alkyl group include methyl group, ethyl group, isopropyl group, propyl group, butyl group, isobutyl group, sec-butyl group, tert-butyl group, pentyl group, sec-pentyl group, tert-pentyl group, isopentyl group, and hexyl group. In an embodiment, the C1-6 alkyl group may be a C1-6 alkyl group other than isobutyl group. Examples of the linear C1-6 alkyl group include methyl group, ethyl group, propyl group, butyl group, pentyl group, and hexyl group. Particular examples of the C1-6 alkyl group (e.g., the linear C1-6 alkyl group) include methyl group and ethyl group. More particular examples of the C1-6 alkyl group (e.g., the linear C1-6 alkyl group) include methyl group.

The term "C6-12 aryl group" refers to an aryl group having a carbon number of 6 to 12. Examples of the C6-12 aryl group include phenyl group, 1-naphthyl group, and 2-naphthyl group. Particular examples of the C6-12 aryl group include phenyl group.

Examples of the substituent that the C1-22 alkyl group of "R₁" may have include C6-12 aryl groups that may have a substituent, aromatic heterocyclic groups that may have a substituent, C3-10 cycloalkyl groups that may have a substituent, C3-10 cycloalkenyl groups that may have a substituent, and non-aromatic heterocyclic groups that may have a substituent.

Examples of the substituent that the C1-6 alkyl group of "R₂" may have include C6-12 aryl groups that may have a substituent, aromatic heterocyclic groups that may have a substituent, C3-10 cycloalkyl groups that may have a substituent, C3-10 cycloalkenyl groups that may have a substituent, non-aromatic heterocyclic groups that may have a substituent, and a C1-7 acyl groups.

Examples of the substituent that the C6-12 aryl groups (including the C6-12 aryl group of "R₂"), the aromatic heterocyclic groups, the C3-10 cycloalkyl groups, the C3-10 cycloalkenyl groups, and the non-aromatic heterocyclic groups may have include hydroxyl group, oxo groups, halogen atoms, C1-7 alkyl groups, C1-7 alkoxy groups, C1-7 alkylenedioxy groups, C1-7 acyl groups, amino group, and C1-7 alkylamino groups. The C6-12 aryl groups (including the C6-12 aryl group of "R₂"), the aromatic heterocyclic groups, the C3-10 cycloalkyl groups, the C3-10 cycloalkenyl groups, and the non-aromatic heterocyclic groups may each have, for example, the same or different 1 to 5 or 1 to 3 substituents selected from these substituents.

Examples of the halogen atoms include fluorine atom, chlorine atom, bromine atom, and iodine atom.

The term "C1-7 alkyl group" refers to an alkyl group having a carbon number of 1 to 7. The C1-7 alkyl group may be linear or branched. Examples of the C1-7 alkyl group include methyl group, ethyl group, isopropyl group, propyl group, butyl group, isobutyl group, sec-butyl group, tert-butyl group, pentyl group, sec-pentyl group, tert-pentyl group, isopentyl group, hexyl group, and heptyl group. Particular examples of the C1-7 alkyl group include methyl group and ethyl group.

The term "C1-7 alkoxy group" refers to an alkoxy group having a carbon number of 1 to 7. The C1-7 alkoxy group may be linear or branched. Examples of the C1-7 alkoxy group include methoxy group, ethoxy group, propoxy group, isopropyloxy group, butoxy group, isobutyloxy group, tert-butyloxy group, pentyloxy group, hexyloxy group, and heptyloxy group. Particular examples of the C1-7 alkoxy group include methoxy group and ethoxy group.

The term "C1-7 alkylenedioxy group" refers to an alkylenedioxy group having a carbon number of 1 to 7. The C1-7 alkylenedioxy group may be linear or branched. Examples of the C1-7 alkylenedioxy group include methylenedioxy group, ethylenedioxy group, trimethylenedioxy group, tetramethylenedioxy group, pentamethylenedioxy group, hexamethylenedioxy group, and heptamethylenedioxy group. Particular examples of the C1-7 alkylenedioxy group include methylenedioxy group and ethylenedioxy group.

The term "C1-7 acyl group" refers to an acyl group having a carbon number of 1 to 7. The C1-7 acyl group may be linear or branched. Examples of the C1-7 acyl group include carboxyl group, carboxamide group, C1-7 alkanoyl groups, benzoyl group, C1-6 alkoxycarbonyl groups, and phenoxycarbonyl group. The term "C1-7 alkanoyl group" refers to an alkanoyl group having a carbon number of 1 to 7. The C1-7 alkanoyl group may be linear or branched. Examples of the C1-7 alkanoyl group include formyl group, acetyl group, propionyl group, butyryl group, isobutyryl group, pentanoyl group, hexanoyl group, and heptanoyl group. The term "C1-6 alkoxycarbonyl group" refers to a carbonyl group having a C1-6 alkoxy group. The term "C1-6 alkoxy group" refers to an alkoxy group having a carbon number of 1 to 6. The C1-6 alkoxy group may be linear or branched. Examples of the C1-6 alkoxycarbonyl group include methoxycarbonyl group, ethoxycarbonyl group, propoxycarbonyl group, isopropoxycarbonyl group, butoxycarbonyl group, isobutoxycarbonyl group, sec-butoxycarbonyl group, and tert-butoxycarbonyl group. Particular examples of the C1-6 alkoxycarbonyl group include methoxycarbonyl group and ethoxycarbonyl group. Particular examples of the C1-7 acyl group that the C6-12 aryl groups (including the C6-12 aryl group of "R₂"), the aromatic heterocyclic groups, the C3-10 cycloalkyl groups, the C3-10 cycloalkenyl groups, and the non-aromatic heterocyclic groups may have include C1-7 alkanoyl groups, benzoyl group, C1-6 alkoxycarbonyl groups, and phenoxycarbonyl group. Particular examples of the C1-7 acyl group that the C1-6 alkyl group of "R₂" may have include carboxyl group, carboxamide group, and C1-6 alkoxycarbonyl groups. More particular examples of the C1-7 acyl group that the C1-6 alkyl group of "R₂" may have include carboxyl group, carboxamide group, methoxycarbonyl group, and ethoxycarbonyl group. The C1-6 alkyl group of "R₂" may be, for example, one in which one of the terminal hydrogen atoms of the alkyl group has been substituted with a C1-7 acyl group. The term "terminal hydrogen atom of an alkyl group" used for the C1-6 alkyl group of "R₂" refers to a hydrogen atom of methyl group in the case of C1 alkyl group (methyl group), and refers to a hydrogen atom attached to a terminal carbon atom on the side not attached to the five-membered ring in the case of a C2 or larger alkyl group.

The term "C1-7 alkylamino group" refers to an amino group mono- or disubstituted with a C1-7 alkyl group. Examples of the C1-7 alkylamino group include methylamino group, ethylamino group, propylamino group, isopropylamino group, butylamino group, isobutylamino group, tert-butylamino group, pentylamino group, isopentylamino group, neopentylamino group, tert-pentylamino group, hexylamino group, and heptylamino group.

The term "aromatic heterocyclic group" refers to an aromatic cyclic group that has carbon atom(s) and 1 to 4 heteroatoms selected from nitrogen, oxygen, and sulfur atoms, wherein the number of atoms constituting the ring is 5 to 14. The aromatic heterocyclic group may be a monocyclic one or a fused-ring one. The aromatic heterocyclic group may be, in particular, a 5- or 6-membered monocyclic aromatic heterocyclic group.

Examples of the monocyclic aromatic heterocyclic group include thienyl groups (e.g., thiophen-2-yl group and thiophen-3-yl group), furyl groups (e.g., furan-2-yl group and furan-3-yl group), pyrrolyl groups (e.g., 2-pyrrol-1-yl group and 3-pyrrol-3-yl group), oxazolyl groups (e.g., oxazol-2-yl group, oxazol-4-yl group, and oxazol-5-yl group), isoxazolyl groups (e.g., isoxazol-3-yl group, isoxazol-4-yl group, and isoxazol-5-yl group), thiazolyl groups (e.g., thiazol-2-yl group, thiazol-4-yl group, and thiazol-5-yl group), isothiazolyl groups (e.g., isothiazol-3-yl group, isothiazol-4-yl group, and isothiazol-5-yl group), imidazolyl groups (e.g., imidazol-1-yl group, 1H-imidazol-2-yl group, and 1H-imidazol-4-yl group), pyrazolyl groups (e.g., 1H-pyrazol-1-yl group, 1H-pyrazol-3-yl group, 2H-pyrazol-3-yl group, and 1H-pyrazol-4-yl group), oxadiazolyl groups (e.g., 1,3,4-oxadiazol-2-yl group, 1,2,3-oxadiazol-4-yl group, 1,2,3-oxadiazol-5-yl group, 1,2,4-oxadiazol-3-yl group, 1,2,4-oxadiazol-5-yl group, and 1,2,5-oxadiazol-3-yl group), thiadiazolyl groups (e.g., 1,3,4-thiadiazol-2-yl group, 1,2,3-thiadiazol-4-yl group, 1,2,3-thiadiazol-5-yl group, 1,2,4-thiadiazol-3-yl group, 1,2,4-thiadiazol-5-yl group, and 1,2,5-thiadiazol-3-yl group), triazolyl groups (e.g., 1,2,4-triazol-3-yl group, 1,2,4-triazol-1-yl group, 1,2,3-triazol-1-yl group, 1,2,3-triazol-2-yl group, and 1,3,4-triazol-1-yl group), tetrazolyl groups (e.g., tetrazol-1-yl group, tetrazol-2-yl group, 1H-tetrazol-5-yl group, and 2H-tetrazol-5-yl group), pyridyl groups (e.g., pyridine-2-yl group, pyridine-3-yl group, and pyridine-4-yl group), pyrimidinyl groups (e.g., pyrimidin-2-yl group, pyrimidin-4-yl group, and pyrimidin-5-yl group), pyridazinyl groups (e.g., pyridazin-3-yl group and pyridazin-4-yl group), pyrazinyl groups (e.g., pyrazin-2-yl group), and triazinyl groups (e.g., 1,3,5-triazinin-2-yl group).

Examples of the fused-ring aromatic heterocyclic group include quinolyl group, isoquinolyl group, quinazolinyl group, quinoxalyl group, phthalazinyl group, cynolinyl group, naphthyridinyl group, indolyl group, benzoimidazolyl group, indolinyl group, benzofuranyl group, benzothienyl group, benzoxazolyl group, benzothiazolyl group, benzodioxynyl group, benzothiazolyl group, tetrahydroquinolyl group, dihydrobenzofuranyl group, dihydrobenzothienyl group, dihydrobenzodioxynyl group, indenothiazolyl group, tetrahydrobenzothiazolyl group, 5,7-dihydropyrrolo[3,4-d]pyrimidinyl group, 6,7-dihydro-5H-cyclopentapyrimidinyl group, imidazo[2,1-b]thiazolyl group, pteridinyl group, and purinyl group.

The term "C3-10 cycloalkyl group" refers to a cyclic saturated hydrocarbon group having a carbon number of 3 to 10. Examples of the C3-10 cycloalkyl group include cyclopropyl group, cyclobutyl group, cyclopentyl group, cyclohexyl group, cycloheptyl group, and cyclooctyl group.

The term "C3-10 cycloalkenyl group" refers to a cyclic partially-unsaturated hydrocarbon group that contains one or more double bonds and has a carbon number of 3 to 10. Examples of the C3-10 cycloalkenyl group include cyclopropenyl group, cyclobutenyl group, cyclopentenyl group, cyclohexenyl group, cycloheptenyl group, and cyclooctenyl group.

The term "non-aromatic heterocyclic group" refers to a non-aromatic cyclic group that has carbon atom(s) and 1 to 4 heteroatoms selected from nitrogen, oxygen, and sulfur atoms, wherein the number of atoms constituting the ring is 3 to 14. The non-aromatic heterocyclic group may be a monocyclic one or a fused-ring one. The non-aromatic heterocyclic group may be, in particular, a 3- to 8-membered monocyclic non-aromatic heterocyclic group. The non-aromatic heterocyclic group may be, in more particular, a 5- or 6-membered monocyclic non-aromatic heterocyclic group. When the non-aromatic heterocyclic group contains a sulfur atom, the sulfur atom may be monoxidized or dioxidized.

Examples of the monocyclic non-aromatic heterocyclic group include oxiranyl group, thiolanyl group, aziridinyl group, azetidinyl group, oxetanyl group, pyrrolidinyl group, tetrahydrofuranyl group, tetrahydrothienyl group, oxazolinyl group, oxazolidinyl group, isoxazolinyl group, isoxazolidinyl group, thiazolinyl group, thiazolidinyl group, isothiazolinyl group, isothiazolidinyl group, imidazolinyl group, imidazolidinyl group, pyrazolinyl group, pyrazolidinyl group, piperidinyl group, piperidino group, pyranyl group, tetrahydropyranyl group, morpholinyl group, morpholino group, thiomorpholinyl group, thiomorpholino group, piperazinyl group, and hexahydro-1,3-oxazinyl group.

Examples of the fused-ring non-aromatic heterocyclic group include isochromanyl group, dihydrobenzopyranyl group, isochromenyl group, chromenyl groups (e.g., 2H-chromenyl group and 4H-chromenyl group), 1,2,3,4-tetrahydroisoquinolyl group, 1,2,3,4-tetrahydroquinolyl group, 2,3-dihydrobenzofuranyl group, and benzo[1,3]dioxolyl group.

Specific examples of the C6-12 aryl groups that may have a substituent (including the C6-12 aryl group that may have a substituent of "R₂") include C6-12 aryl groups (e.g., phenyl group and naphthyl group) that may be substituted with the same or different 1 to 5 (e.g., 1 to 3) substituents selected from hydroxyl group and C1-7 alkoxy groups (e.g., methoxy group and ethoxy group). More specific examples of the C6-12 aryl groups that may have a substituent (including the C6-12 aryl group that may have a substituent of "R₂") include 4-hydroxyphenyl group, 4-methoxyphenyl group, 3,4-dihydroxyphenyl group, 4-hydroxy-3-methoxyphenyl group, 3-hydroxy-4-methoxyphenyl group, and 2,4-dihydroxyphenyl group. Particular examples of the C6-12 aryl groups that may have a substituent (including the C6-12 aryl group that may have a substituent of "R₂") include 4-hydroxyphenyl group and 3,4-dihydroxyphenyl group.

Specific examples of the aromatic heterocyclic groups that may have a substituent include pyridyl groups (e.g., pyridine-2-yl group, pyridine-3-yl group, and pyridine-4-yl group).

Specific examples of the C3-10 cycloalkyl groups that may have a substituent include cyclohexyl group and cyclopentyl group.

Specific examples of the C3-10 cycloalkenyl groups that may have a substituent include cyclohexenyl group.

Specific examples of the non-aromatic heterocyclic groups that may have a substituent include non-aromatic heterocyclic groups (e.g., pyranyl group and tetrahydropyranyl group) that may be substituted with the same or different 1 to 5 (e.g., 1 to 3) substituents selected from hydroxyl group and oxo groups. More specific examples of the non-aromatic heterocyclic groups that may have a substituent include tetrahydro-2H-pyran-2-yl group and 5-hydroxy-4-oxo-4H-pyran-2-yl group. Particular examples of the non-aromatic heterocyclic groups that may have a substituent include 5-hydroxy-4-oxo-4H-pyran-2-yl group.

Specific examples of the C1-22 alkyl group that may have a substituent of "R₁" include C1-12 alkyl groups that may be substituted with a C6-12 aryl group that may have a substituent and C1-12 alkyl groups that may be substituted with a non-aromatic heterocyclic group that may have a substituent. More specific examples of the C1-22 alkyl group that may have a substituent of "R₁" include C1-12 alkyl groups (preferably, C1-6 alkyl groups), C1-6 alkyl groups substituted with a C6-12 aryl group that may have a substituent, and C1-6 alkyl groups substituted with a non-aromatic heterocyclic group that may have a substituent. Further specific examples of the C1-22 alkyl group that may have a substituent of "R₁" include methyl group, ethyl group, propyl group, isopropyl group, butyl group, hexyl group, octyl group, dodecyl group, 4-hydroxyphenylethyl group (e.g., 2-(4-hydroxyphenyl)ethyl group), 3,4-dihydroxyphenylethyl group (e.g., 2-(3,4-dihydroxyphenyl)ethyl group), and (5-hydroxy-4-oxo-4H-pyran-2-yl)methyl group.

Specific examples of the C1-6 alkyl group that may have a substituent of "R₂" include C1-6 alkyl groups that may be substituted with a C6-12 aryl group that may have a substituent, C1-6 alkyl groups that may be substituted with a non-aromatic heterocyclic group that may have a substituent, and C1-6 alkyl groups that may be substituted with a C1-7 acyl group. More specific examples of the C1-6 alkyl group that may have a substituent of "R₂" include C1-6 alkyl groups, C1-6 alkyl groups substituted with a C6-12 aryl group that may have a substituent, C1-6 alkyl groups substituted with a non-aromatic heterocyclic group that may have a substituent, and C1-6 alkyl groups substituted with a C1-7 acyl group. Further specific examples of the C1-6 alkyl group that may have a substituent of "R₂" include methyl group, ethyl group, isopropyl group, hexyl group, 4-hydroxyphenylmethyl group, 4-hydroxyphenylethyl group (e.g., 2-(4-hydroxyphenyl)ethyl group), 3,4-dihydroxyphenylethyl group (e.g., 2-(3,4-dihydroxyphenyl)ethyl group), (5-hydroxy-4-oxo-4H-pyran-2-yl)methyl group, C1-6 alkyl groups substituted with a carboxyl group (e.g., 2-carboxyethyl group), C1-6 alkyl groups substituted with a carboxamide group (e.g., 2-carboxamidoethyl group), C1-6 alkyl groups substituted with a methoxycarbonyl group (e.g., 2-methoxycarbonylethyl group), and C1-6 alkyl groups substituted with an ethoxycarbonyl group (e.g., 2-ethoxycarbonylethyl group). Particular examples of the C1-6 alkyl group that may have a substituent of "R₂" include methyl group and 4-hydroxyphenylmethyl group. More particular examples of the C1-6 alkyl group that may have a substituent of "R₂" include methyl group.

In an embodiment, the C1-6 alkyl group that may have a substituent of "R₂" may be a C1-6 alkyl group that may have a substituent, but that is other than isobutyl group. Also, in an embodiment, the C1-6 alkyl group that may have a substituent of "R₂" may be a C1-6 alkyl group that may have a substituent, but that is other than isobutyl group that may have a substituent. The C1-6 alkyl group that may have a substituent of "R₂" may be, in particular, a linear C1-6 alkyl group that may have a substituent.

Examples of the linear C1-6 alkyl group that may have a substituent of "R₂" include the above exemplified C1-6 alkyl groups that may have a substituent, but which C1-6 alkyl groups each are a linear C1-6 alkyl group. That is, specific examples of the linear C1-6 alkyl group that may have a substituent of "R₂" include linear C1-6 alkyl groups that may be substituted with a C6-12 aryl group that may have a substituent, linear C1-6 alkyl groups that may be substituted with a non-aromatic heterocyclic group that may have a substituent, and linear C1-6 alkyl groups that may be substituted with a C1-7 acyl group. More specific examples of the linear C1-6 alkyl group that may have a substituent of "R₂" include linear C1-6 alkyl groups, linear C1-6 alkyl groups substituted with a C6-12 aryl group that may have a substituent, linear C1-6 alkyl groups substituted with a non-aromatic heterocyclic group that may have a substituent, and linear C1-6 alkyl groups substituted with a C1-7 acyl group. Further specific examples of the linear C1-6 alkyl group that may have a substituent of "R₂" include methyl group, ethyl group, hexyl group, 4-hydroxyphenylmethyl group, 4-hydroxyphenylethyl group (e.g., 2-(4-hydroxyphenyl)ethyl group), 3,4-dihydroxyphenylethyl group (e.g., 2-(3,4-dihydroxyphenyl)ethyl group), (5-hydroxy-4-oxo-4H-pyran-2-yl)methyl group, linear C1-6 alkyl groups substituted with a carboxyl group (e.g., 2-carboxyethyl group), linear C1-6 alkyl groups substituted with a carboxamide group (e.g., 2-carboxamidoethyl group), linear C1-6 alkyl groups substituted with a methoxycarbonyl group (e.g., 2-methoxycarbonylethyl group), and linear C1-6 alkyl groups substituted with an ethoxycarbonyl group (e.g., 2-ethoxycarbonylethyl group). Particular examples of the linear C1-6 alkyl group that may have a substituent of "R₂" include methyl group and 4-hydroxyphenylmethyl group. More particular examples of the linear C1-6 alkyl group that may have a substituent of "R₂" include methyl group.

The active ingredient may be, for example, a 2R compound, a 2S compound, or a combination thereof (i.e., a diastereomeric mixture of the 2R and 2S compounds). The notation "2RS" in the name of a compound indicates that the compound is a combination of the 2R and 2S compounds (i.e., a diastereomeric mixture of the 2R and 2S compounds). The ratio of the 2R and 2S compounds in the diastereomeric mixture is not particularly limited. The ratio of the 2R or 2S compound in the diastereomeric mixture, for example, may be 1% or more, 3% or more, 5% or more, 10% or more, 20% or more, 30% or more, 40% or more, 50% or more, 60% or more, 70% or more, or 80% or more, may be 99% or less, 97% or less, 95% or less, 90% or less, 80% or less, 70% or less, 60% or less, 50% or less, 40% or less, 30% or less, or 20% or less, or may be within a range defined as a non-contradictory combination thereof, in molar ratio to the total amount of the 2R and 2S compounds. The ratio of the 2R or 2S compound in the diastereomeric mixture may be, specifically, for example, 1 to 99%, 10 to 90%, 20 to 80%, 30 to 70%, or 40 to 60%, in molar ratio to the total amount of the 2R and 2S compounds. When the 2R or 2S compound is selected as the active ingredient, it is sufficient that the 2R or 2S compound is used as the active ingredient, and it does not preclude further use of the 2S or 2R compound in combination.

Specific examples of the active ingredient include (2RS,4R)-2-methyl-2,4-thiazolidinedicarboxylic acid 2-ethyl ester, (2RS,4R)-2-methyl-2,4-thiazolidinedicarboxylic acid 2-methyl ester, (2RS,4R)-2-methyl-2,4-thiazolidinedicarboxylic acid 2-i-propyl ester, (2RS,4R)-2-methyl-2,4-thiazolidinedicarboxylic acid 2-n-butyl ester, (2RS,4R)-2-methyl-2,4-thiazolidinedicarboxylic acid 2-t-butyl ester, (2RS,4R)-2-methyl-2,4-thiazolidinedicarboxylic acid 2-n-octyl ester, (2RS,4R)-2,4-thiazolidinedicarboxylic acid 2-ethyl ester, and (2RS,4R)-2-(4-hydroxyphenyl)methyl-2,4-thiazolidinedicarboxylic acid 2-methyl ester, and the 2R and 2S compounds thereof. Particular examples of the active ingredient include (2RS,4R)-2-methyl-2,4-thiazolidinedicarboxylic acid 2-ethyl ester, (2RS,4R)-2-methyl-2,4-thiazolidinedicarboxylic acid 2-methyl ester, (2RS,4R)-2-methyl-2,4-thiazolidinedicarboxylic acid 2-i-propyl ester, (2RS,4R)-2,4-thiazolidinedicarboxylic acid 2-ethyl ester, and (2RS,4R)-2-(4-hydroxyphenyl)methyl-2,4-thiazolidinedicarboxylic acid 2-methyl ester, and 2R and 2S compounds thereof. More particular examples of the active ingredient include (2RS,4R)-2-methyl-2,4-thiazolidinedicarboxylic acid 2-ethyl ester, and the 2R and 2S compounds thereof.

The active ingredient may be used as a free compound, a salt thereof, or a combination of them. That is, the term "active ingredient" may mean the active ingredient in the form of free compound, a salt thereof, or a combination of them, unless otherwise stated. In other words, the term "compound shown by the formula (I)" may mean the compound shown by the formula (I) in the form of free compound, a salt thereof, or a combination of them, unless otherwise stated. The salt is not particularly limited, so long as it is usable for culture of animal cells. For example, examples of salts for acidic groups such as carboxyl group include ammonium salts, salts with alkali metals such as sodium and potassium, salts with alkaline earth metals such as calcium and magnesium, aluminum salts, zinc salts, salts with organic amines such as triethylamine, ethanolamine, morpholine, pyrrolidine, piperidine, piperazine, and dicyclohexylamine, and salts with basic amino acids such as arginine and lysine. Also, examples of salts for basic groups such as amino group include salts with inorganic acids such as hydrochloric acid, sulfuric acid, phosphoric acid, nitric acid, and hydrobromic acid, salts with organic carboxylic acids such as acetic acid, citric acid, benzoic acid, maleic acid, fumaric acid, tartaric acid, succinic acid, tannic acid, butyric acid, hibenzic acid, pamoic acid, enanthic acid, decanoic acid, teoclic acid, salicylic acid, lactic acid, oxalic acid, mandelic acid, and malic acid, and salts with organic sulfonic acid such as methanesulfonic acid, benzenesulfonic acid, and p-toluenesulfonic acid. As the salt, one kind of salt may be used, or two or more kinds of salts may be used in combination. The active ingredient (e.g., free compound or salt) may include both non-hydrate and hydrate, unless otherwise stated.

The active ingredient may be, for example, highly stable in a culture medium. The phrase "highly stable in a culture medium" may mean, for example, that when a culture medium containing 58 mM of the active ingredient is prepared and stored at 5°C in dark conditions, no precipitation of crystals is visibly observed for 15 days or longer, 20 days or longer, 25 days or longer, or 30 days or longer.

As the active ingredient, a commercially available product may be used, or one appropriately prepared and obtained may be used. Methods for producing the active ingredient are not particularly limited, and for example, known methods can be used. The active ingredient can be produced by, for example, a chemical synthesis (JP patent No. 5439849).

The active ingredient (compound I) can be easily produced, specifically, for example, from a compound II (pyruvate derivative) and a compound III (L-cysteine) by the following reaction. The structural formula of the compound II is also referred to as "formula (II)". In other words, the compound II is a pyruvic acid derivative shown by the formula (II). "R₁" and "R₂" in the compounds I and II are the same as "R₁" and "R₂" in the formula (I), respectively. Upon production of the active ingredient, components that can form a salt each may be used as a free compound, a salt thereof, or a combination of them. That is, when the compound II can form a salt, the term "compound II" or "pyruvate derivative" may mean the compound II in the form of free compound, a salt thereof, or a combination of them, unless otherwise stated. Also, the term "compound III" or "L-cysteine" may mean the compound III in the form of free compound, a salt thereof, or a combination of them, unless otherwise stated. For the salt, the descriptions concerning the salt of the active ingredient can be similarly applied. Furthermore, these components (e.g., free compounds or salts) each may include both non-hydrate and hydrate, unless otherwise stated. The reaction between the compound II and the compound III can be carried out in, for example, a suitable solvent such as water, aqueous buffer, alcohol, or hydrous alcohol. The generated active ingredient (compound I) can be isolated and purified by, for example, a known separation method such as vacuum concentration, solvent extraction, crystallization, transdissolution, or chromatography. The structure of the generated active ingredient (compound I) can be confirmed by, for example, a known analytical means such as

### NMR

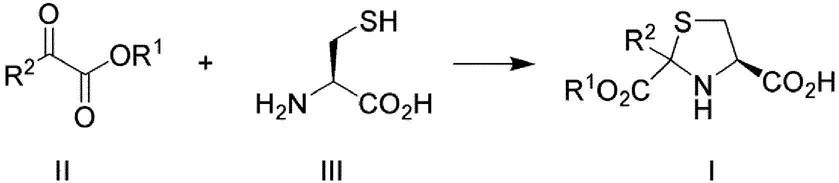

In an embodiment, the reaction between the compound II and the compound III can be carried out in a hydrous medium to thereby form crystals of the active ingredient, and the crystals can be collected to thereby obtain the active ingredient.

That is, the present invention provides a method for producing the active ingredient, the method comprising:
reacting the compound II and the compound III in a hydrous medium to thereby form crystals of the active ingredient; and
collecting the crystals.

Hereinafter, this method is also referred to as "direct crystallization method".

In cases of producing the active ingredient by the direct crystallization method, "R₁" may be, in particular, a C1-C6 alkyl group. In cases of producing the active ingredient by the direct crystallization method, "R₁" may be, in more particular, methyl group or ethyl group. In cases of producing the active ingredient by the direct crystallization method, "R₂" may be, in particular, methyl group.

The reaction between the compound II and the compound III can proceed by making these compounds coexist in a hydrous medium. For example, the compound III may be dissolved or suspended in the hydrous medium, and the compound II may be added dropwise to the resulting solution or suspension. The use amounts of the compound II and the compound III can be appropriately set. The use amount of the compound II may be, for example, 0.5 to 2 moles, or 1 to 2 moles, per mole of the use amount of the compound III. The hydrous medium is not particularly limited, so long as it is a medium that contains water, and crystals of the active ingredient are generated in the medium (i.e., the generated active ingredient is crystallized in the medium). The hydrous medium may be water itself, or may be a mixture of water and other component(s). Examples of such a mixture include aqueous buffers and hydrous alcohols. The pH of the hydrous medium may be adjusted. For example, an acid and/or an alkali may be used to adjust the pH. The water content in the hydrous medium may be, for example, 70%(w/w) or more, 80%(w/w) or more, 90%(w/w) or more, 95%(w/w) or more, 97%(w/w) or more, or 99%(w/w) or more. The reaction pH may be, for example, 5 to 9, or 6 to 8. The reaction temperature, for example, may be 0°C or higher, 10°C or higher, 20°C or higher, 30°C or higher, 40°C or higher, or 50°C or higher, may be 80°C or lower, 70°C or lower, 60°C or lower, 50°C or lower, 40°C or lower, or 30°C or lower, or may be within a range defined as a non-contradictory combination thereof. The reaction temperature may be, specifically, for example, 10°C to 40°C, or room temperature (e.g., approximately 25°C). The reaction period, for example, may be 1 hour or longer, 5 hours or longer, 10 hours or longer, 15 hours or longer, 20 hours or longer, or 25 hours or longer, may be 36 hours or shorter, 30 hours or shorter, 24 hours or shorter, 20 hours or shorter, or 15 hours or shorter, or may be within a range defined as a non-contradictory combination thereof. The reaction period may be, specifically, for example, 1 to 24 hours. The reaction may be carried out, for example, with stirring or shaking. By carrying out the reaction between the compound II and the compound III, crystals of the active ingredient are formed in the hydrous medium.

Collection of the crystals of the active ingredient can be carried out by, for example, a known solid-liquid separation means such as filtration or centrifugal separation.

By the direct crystallization method, for example, the active ingredient mainly containing the 2S or 2R compound may be obtained. By the direct crystallization method, in particular, the active ingredient mainly containing the 2S compound may be obtained. The ratio of the 2S or 2R compound in the active ingredient obtained by the direct crystallization method may be, for example, 60% or more, 70% or more, or 80% or more, in molar ratio.

When two or more components are selected as the active ingredient, the "amount" or "concentration" of the active ingredient may refer to the total amount or total concentration of the selected components, unless otherwise stated.

### <2> Method of the present invention

The method of the present invention is a method for culturing animal cells, comprising culturing the animal cells in a culture medium, wherein the culturing is carried out in the presence of the active ingredient.

In an embodiment, an objective substance may be produced by culturing the animal cells. That is, when the animal cells have an objective substance-producing ability, an objective substance can be produced by culturing the animal cells. That is, an embodiment of the method of the present invention is a method for producing an objective substance, comprising culturing animal cells having an objective substance-producing ability in a culture medium, and collecting the objective substance, wherein the culturing is carried out in the presence of the active ingredient.

The objective substance is not particularly limited, so long as it can be produced by animal cells. Examples of the objective substance include proteins. A protein to be produced as the objective substance is also referred to as "objective protein".

The animal cells are not particularly limited. The animal cells can be chosen according to various conditions such as the use purpose of the animal cells. For example, when using the animal cells for production of the objective protein, the animal cells are not particularly limited, so long as they can express the objective protein. The animal cells each are also referred to as "host", "expression host", or "host cell". Examples of the animal include mammals, birds, and amphibians. Particular examples of the animal include mammals. Examples of the mammals include rodents and primates. Examples of the rodents include hamster, mouse, rat, and guinea pig. Examples of the hamster include Chinese hamster. Examples of the primates include human, monkey, and chimpanzee. Examples of the monkey include African green monkey. Examples of the birds include chicken. Examples of the amphibians include *Xenopus laevis.* The tissue or cell from which the animal cells is derived is not particularly limited. Examples of the tissue or cell from which the animal cells is derived include ovary, kidney, adrenal gland, tongue epithelium, olfactory epithelium, pineal body, thyroid gland, and melanocyte. Examples of the cells of Chinese hamster include Chinese hamster ovary-derived cell line (CHO). Specific examples of CHO include CHO-DG44, CHO-K1, CHO DUX (DHFR-), CHO-S, and CHO-MK Examples of the cells of human include human embryonic kidney cell-derived cell line (HEK). Specific examples of HEK include HEK293 and HEK293T. Examples of the cells of African green monkey include African green monkey kidney cell-derived cell line (COS). Specific examples of COS include COS-1. Specific examples of the cells of *Xenopus laevis* include *Xenopus laevis* oocyte.

The term "animal cells having an objective substance-producing ability" refers to animal cells having an ability to produce the objective substance. The term "animal cells having an objective substance-producing ability" may specifically refer to animal cells having an ability to, when being cultured in a culture medium, generate the objective substance (e.g., express the objective protein) and accumulate the same in a culture broth to such an extent that the objective substance can be collected therefrom. The term "accumulation in a culture broth" may specifically refer to accumulation in a culture medium, on a cell surface layer, in cells, or in/on a combination thereof. Accumulation of the objective substance outside the cells, e.g., in a culture medium or on a cell surface layer, is also referred to as "secretion" or "secretory production" of the objective substance. That is, the animal cells may have a secretory production ability of the objective substance (i.e., an ability to produce the objective substance by secretory production). The accumulation amount of the objective substance may be, for example, 10 µg/L or more, 1 mg/L or more, 100 mg/L or more, or 1 g/L or more, in terms of the accumulation amount in a culture broth. The animal cells may have a production ability of one kind of the objective substance or two or more kinds of the objective substance.

The animal cells may inherently have an objective substance-producing ability, or may have been modified so as to have an objective substance-producing ability. The animal cells may also have been modified so that an objective substance-producing ability inherently possessed by them is enhanced. The animal cells having an objective substance-producing ability can be obtained by imparting an objective substance-producing ability to such animal cells as described above, or enhancing an objective substance-producing ability of such animal cells as described above. For example, an objective protein-producing ability can be imparted or enhanced by introduction of a gene encoding the objective protein. The gene encoding the objective protein is also referred to as "objective protein gene".

The objective protein is not particularly limited, so long as it can be expressed by using animal cells as a host. The objective protein may be a protein derived from the host, or may be a heterologous protein. The term "heterologous protein" refers to an exogenous protein relative to the host producing that protein (i.e., the animal cells having an objective protein-producing ability). The objective protein may be, for example, a naturally-present protein, a modified protein thereof, or a protein of which the amino acid sequence is artificially designed. The objective protein may be, for example, a protein derived from a microorganism, a protein derived from a plant, a protein derived from an animal, or a protein derived from a virus. The objective protein may particularly be a derived from human. The objective protein may be a monomeric protein or a multimeric protein. The objective protein may be a secretory protein or a non-secretory protein. The term "protein" also include those called peptides, such as oligopeptides and polypeptides.

Specific examples of the objective protein include enzymes, physiologically active proteins, receptor proteins, antigenic proteins, and other proteins.

Examples of the enzymes include cellulase, transglutaminase, protein glutaminase, isomaltodextranase, protease, endopeptidase, exopeptidase, aminopeptidase, carboxypeptidase, collagenase, and chitinase.

Examples of the physiologically active proteins include growth factors, hormones, cytokines, and antibody-related molecules.

Examples of the growth factors include epidermal growth factor (EGF), insulin-like growth factor-1 (IGF-1), transforming growth factor (TGF), nerve growth factor (NGF), brain-derived neurotrophic factor (BDNF), vascular endothelial growth factor (VEGF), granulocyte-colony stimulating factor (G-CSF), granulocyte-macrophage-colony stimulating factor (GM-CSF), platelet-derived growth factor (PDGF), erythropoietin (EPO), thrombopoietin (TPO), acidic fibroblast growth factor (aFGF or FGF1), basic fibroblast growth factor (bFGF or FGF2), keratinocyte growth factor (KGF-1 or FGF7, and, KGF-2 or FGF10), hepatocyte growth factor (HGF), stem cell factor (SCF), and Activin. Examples of Activin include Activin A, C, and E.

Examples of the hormones include insulin, glucagon, somatostatin, human growth hormone (hGH), parathyroid hormone (PTH), calcitonin, and exenatide.

Examples of the cytokines include interleukins, interferons, and tumor necrosis factors (TNFs).

Furthermore, a physiologically active protein may be an intact protein, or may be a part of a protein. Examples of a part of a protein include, for example, a part having physiological activity. Specific examples of a part having physiological activity include, for example, Teriparatide, a physiologically active peptide consisting of the N-terminal 34 amino acid residues of parathyroid hormone (PTH).

The term "antibody-related molecule" may refer to a protein containing a molecular species consisting of a single domain or a combination of two or more domains selected from the domains constituting a complete antibody. Examples of the domains constituting a complete antibody include heavy chain domains VH, CH1, CH2, and CH3, and light chain domains VL and CL. The antibody-related molecule may be a monomeric protein, or may be a multimeric protein, so long as it contains the above-mentioned molecular species. When the antibody-related molecule is a multimeric protein, it may be a homo-multimer consisting of a single kind of subunit, or may be a hetero-multimer consisting of two or more kinds of subunits. Specific examples of the antibody-related molecules include complete antibody, Fab, F(ab'), F(ab')₂, Fc, dimer consisting of a heavy chain (H chain) and a light chain (L chain), Fc-fusion protein, heavy chain (H chain), light chain (L chain), light chain Fv (scFv), sc(Fv)₂, disulfide-bonded Fv (sdFv), diabody, and VHH fragment (Nanobody (registered trademark)). More specific examples of the antibody-related molecules include Trastuzumab, Adalimumab, and Nivolumab.

Examples of the receptor proteins include receptor proteins for physiologically active proteins and other physiologically active substances. Examples of the other physiologically active substances include neurotransmitters such as dopamine. A receptor protein may be an orphan receptor of which the corresponding ligand is not known.

The antigen proteins are not particularly limited, so long as they can induce an immune response. An antigen protein can be appropriately selected depending on, for example, the intended object of the immune response. An antigen protein can be used as, for example, a vaccine.

Examples of other proteins include Liver-type fatty acid-binding protein (LFABP), fluorescent proteins, immunoglobulin-binding proteins, albumin, fibroin-like proteins, and extracellular proteins. Examples of the fluorescent proteins include Green Fluorescent Protein (GFP). Examples of the immunoglobulin-binding proteins include Protein A, Protein G, and Protein L. Examples of albumin include human serum albumin. Examples of the fibroin-like proteins include those disclosed in WO2017/090665 and WO2017/171001.

Examples of the extracellular protein include fibronectin, vitronectin, collagen, osteopontin, laminin, and partial sequences thereof. Laminin is a protein having a heterotrimeric structure consisting of an α chain, a β chain, and a γ chain. Examples of laminin include laminin of mammals. Examples of the subunit chains of laminin (i.e., α, β, and γ chains) include 5 kinds of α chains (α1 to α5), 3 kinds of β chains (β1 to β3), and 3 kinds of γ chains (γ1 to γ3). Laminin constitutes various isoforms depending on combinations of these subunits. Specific examples of laminin include, for example, laminin 111, laminin 121, laminin 211, laminin 213, laminin 221, laminin 311, laminin 321, laminin 332, laminin 411, laminin 421, laminin 423, laminin 511, laminin 521, and laminin 523. Examples of the partial sequence of laminin include laminin E8, which is an E8 fragment of laminin. Laminin E8 is a protein having a heterotrimeric structure consisting of an E8 fragment of α chain (α chain E8), an E8 fragment of β chain (β chain E8), and an E8 fragment of γ chain (γ chain E8). The subunit chains of laminin E8 (i.e., α chain E8, β chain E8, and γ chain E8) are also collectively referred to as "E8 subunit chains". Examples of the E8 subunit chains includes E8 fragments of the laminin subunit chains exemplified above. Laminin E8 constitutes various isoforms depending on combinations of these E8 subunit chains. Specific examples of laminin E8 include, for example, laminin 111E8, laminin 121E8, laminin 211E8, laminin 221E8, laminin 332E8, laminin 421E8, laminin 411E8, laminin 511E8, and laminin 521E8.

The objective protein may be, for example, a protein having any of known or natural amino acid sequences of such proteins as described above. The objective protein may also be, for example, a variant of a protein having any of known or natural amino acid sequences of such proteins as described above. Examples of the variant include a protein having any of known or natural amino acid sequences including substitution, deletion, insertion, or addition of one or several amino acid residues at one or several positions. The number meant by the term "one or several" used above may specifically be, for example, 1 to 50, 1 to 40, or 1 to 30, preferably 1 to 20, more preferably 1 to 10, still more preferably 1 to 5, particularly preferably 1 to 3. Examples of the variant also include a protein having an amino acid sequence showing an identity of, for example, 50% or more, 65% or more, or 80% or more, preferably 90% or more, more preferably 95% or more, still more preferably 97% or more, particularly preferably 99% or more, to the total amino acid sequence of any of known or natural amino acid sequences. A protein specified with the type of organism from which the protein is derived is not limited to proteins themselves found in that organism, and shall also include proteins having any of the amino acid sequences of proteins found in that organism and variants thereof. Such variants may or may not be found in that organism. That is, for example, the term "protein derived from human" is not limited to proteins themselves found in human, and shall also include proteins having any of the amino acid sequences of proteins found in human and variants thereof.

The term "identity" between amino acid sequences means an identity between the amino acid sequences calculated by blastp with default scoring parameters (i.e., Matrix, BLOSUM62; Gap Costs, Existence = 11, Extension = 1; Compositional Adjustments, Conditional compositional score matrix adjustment), unless otherwise stated.

The objective protein gene is not particularly limited, so long as it encodes such an objective protein as described above. The objective protein gene may be, for example, a gene having any of known or natural nucleotide sequences of genes encoding such proteins as described above. The objective protein gene may also be, for example, a variant of a gene having any of known or natural nucleotide sequences of genes encoding such proteins as described above. The objective protein gene may have been modified, for example, so as to encode a protein having such a variant sequence as exemplified above. In the objective protein gene, any codon(s) may be replaced with respective equivalent codon(s) thereof. For example, the objective protein gene may have been modified so as to have optimal codons according to the codon usage frequency of the host cell.

In the present invention, the term "gene" is not limited to DNA, but may include any polynucleotide, so long as it encodes a corresponding expression product. That is, the term "objective protein gene" may mean any polynucleotide encoding the objective protein. The objective protein gene may be DNA, RNA, or a combination thereof. The objective protein gene may be single-stranded or double-stranded. The objective protein gene may be single-stranded DNA or single-stranded RNA. The objective protein gene may be double-stranded DNA, double-stranded RNA, or a hybrid strand consisting of a DNA strand and an RNA strand. The objective protein gene may contain both a DNA residue and an RNA residue in a single polynucleotide chain. The objective protein gene may or may not contain an intron. The embodiment of the objective protein gene can be appropriately chosen according to various conditions such as means for expressing the objective protein.

The expression "having a (nucleotide or amino acid) sequence" means "comprising the (nucleotide or amino acid) sequence" unless otherwise stated, and also includes cases of "consisting of the (nucleotide or amino acid) sequence".

The objective protein is expressed from the objective protein gene. That is, the animal cells having an objective protein-producing ability have the objective protein gene. Specifically, the animal cells having an objective protein-producing ability have the objective protein gene so that the gene can be expressed. Incidentally, it is sufficient that the animal cells having an objective protein-producing ability have the objective protein gene till the objective protein is expressed to a desired extent. That is, the animal cells having an objective protein-producing ability may or may not have the objective protein gene after expression of the objective protein. The terms "expression of the objective protein gene" and "expression of the objective protein" may be used synonymously with each other.

The objective protein gene can be obtained by cloning from an organism having the objective protein gene. For cloning, for example, nucleotides containing the gene, such as genomic DNA and cDNA, can be used. The objective protein gene can also be obtained by chemical synthesis (Gene, 60(1), 115-127 (1987)).

The obtained objective protein gene can be used as it is, or after being modified as required. That is, the objective protein gene can be modified to obtain a variant thereof. A gene can be modified by a known technique. For example, an objective mutation can be introduced into an objective site of DNA by the site-specific mutation method. Examples of the site-specific mutation method include the method utilizing PCR (Higuchi, R., 61, in PCR Technology, Erlich, H.A. Eds., Stockton Press (1989); Carter, P., Meth. in Enzymol., 154, 382 (1987)), and the method utilizing phage (Kramer, W. and Frits, H.J., Meth. in Enzymol., 154, 350 (1987); Kunkel, T.A. et al., Meth. in Enzymol., 154, 367 (1987)). Alternatively, a variant of the objective protein gene may also be obtained directly by chemical synthesis.

Modes of introducing objective protein gene into the host cell are not particularly limited. It is sufficient that the objective protein gene is harbored by the host cell in such a manner that it can be expressed. Specifically, for example, in cases of introducing the objective protein gene in the form requiring transcription, such as DNA, it is sufficient that the objective protein gene is harbored by the host cell in such a manner that it can be expressed under control of a promoter that functions in the host cell. In the host cell, the objective protein gene may be present outside the chromosome, or may have been integrated into the chromosome. In cases of introducing two or more genes, it is sufficient that the genes are harbored by the host cell in such a manner that they each can be expressed.

The promoter for expressing the objective protein gene is not particularly limited so long as it functions in the host cell. The term "promoter that functions in host cell" refers to a promoter that shows a promoter activity in the host cell. The promoter may be a promoter derived from the host cell, or a heterogenous promoter. The promoter may be the native promoter of the objective protein gene, or a promoter of another gene. The promoter may also be a promoter stronger than the native promoter of the objective protein gene. Examples of promoters that function in animal cells include SV40 promoter, EF1a promoter, RSV promoter, CMV promoter, and SRalpha promoter. As the promoter, a highly-active type of an existing promoter may also be obtained and used by using various reporter genes. Methods for evaluating the strength of promoters and examples of strong promoters are described in the paper of Goldstein et al. (Prokaryotic Promoters in Biotechnology, Biotechnol. Annu. Rev., 1, 105-128 (1995)), and so forth.

The objective protein gene can be introduced into the host cell by, for example, using a vector containing the gene. The vector containing the objective protein gene is also referred to as "expression vector for the objective protein gene". The expression vector for the objective protein gene can be constructed by, for example, ligating a DNA fragment containing the objective protein gene with a vector. By introducing the expression vector for the objective protein gene into the host cell, the gene can be introduced into the host cell. The vector may contain a marker such as a drug resistance gene. Furthermore, the vector may contain an expression control sequence, such as a promoter, for expressing the inserted gene. The vector can be appropriately selected according to various conditions such as the type of the host cell and the mode of introducing the objective protein gene. Examples of vectors usable for gene introduction into animal cells include plasmid vectors and viral vectors. Examples of the viral vectors include, for example, retroviral vectors and adenoviral vectors. Examples of the plasmid vectors include, for example, pcDNA series vectors (e.g., pcDNA3.1; Thermo Fisher Scientific), pBApo-CMV series vectors (TAKARA BIO), and pCI-neo (Promega). Depending on the type and structure of the vector, the vector can be integrated into the chromosome of the host cell, autonomously replicated outside the chromosome of the host cell, or temporarily held outside the chromosome of the host cell. For example, a vector having a viral replication origin, such as SV40 replication origin, can be autonomously replicated outside the chromosome in animal cells. Specifically, for example, the pcDNA series vectors have the SV40 replication origin, and hence can be autonomously replicated outside the chromosome in the host cell expressing the SV40 large T antigen, such as COS-1 and HEK293T.

Alternatively, the objective protein gene can be introduced into the host cell by, for example, introducing a nucleotide fragment containing the gene into the host cell. Examples of such a nucleotide fragment include linear DNA and linear RNA. Examples of the linear RNA include, for example, mRNA and cRNA.

Methods for introducing a nucleotide such as a vector and nucleotide fragment into the host cell can be appropriately selected according to various conditions such as the type of the host cell. Examples of methods for introducing a nucleotide such as a vector and nucleotide fragment into animal cells include the DEAE dextran method, the calcium phosphate method, the lipofection method, the electroporation method, and the microinjection method. When the vector is a viral vector, the vector can be introduced into the host cell by infecting the host cell with the vector (virus).

Furthermore, cells inherently having the objective protein gene may be modified so that the expression of the objective protein gene is increased, and then used. The expression "the expression of a gene is increased" means that the expression amount of the gene per cell is increased as compared with that of a non-modified cell. The term "non-modified cell" used herein refers to a control cell that has not been modified so that the expression of an objective gene is increased. Examples of the non-modified cell include a wild-type cell and a cell from which the host cell is obtained though modification. Examples of methods for increasing the expression of the objective protein gene include increasing the copy number of the objective protein gene, and improving the transcription efficiency and/or translation efficiency of the objective protein gene. The copy number of the objective protein gene can be increased by introducing the objective protein gene into the host cell. Introduction of the objective protein gene can be carried out as described above. The objective protein gene to be introduced may be one derived from the host cell, or a heterogenous one. The transcription efficiency and/or translation efficiency of the objective protein gene can be improved by modifying an expression control sequence of the gene, such as a promoter. For example, the transcription efficiency of the objective protein gene can be improved by replacing the promoter of the objective protein gene with a stronger promoter.

The culture of the animal cells is carried out in the presence of the active ingredient. That is, the culture of the animal cells is carried out by using the active ingredient.

By using the active ingredient, for example, the culture performance of the animal cells may be improved. That is, by culturing the animal cells in the presence of the active ingredient, the culture performance of the animal cells may be improved as compared with a case of culturing the animal cells in the absence of the active ingredient. Examples of the case of culturing the animal cells in the absence of the active ingredient include a case of culturing the animal cells under the same conditions with those for the case of culturing the animal cells in the presence of the active ingredient except that the active ingredient is not used. By using the active ingredient, in particular, the culture performance of the animal cells during a period where the animal cells are present in the culture medium at a sufficient viable cell density may be improved. Examples of the improvement of the culture performance of the animal cells include improvement of the proliferation of the animal cells, improvement of the survival rate of the animal cells, improvement of production of the objective substance by the animal cells, and elongation of the culture period of the animal cells. Examples of the elongation of the culture period of the animal cells include elongation of a period where the proliferation of the animal cells continues, elongation of a period where the animal cells are maintained (i.e., the animal cells survive), and elongation of a period where production of the objective substance such as the objective protein by the animal cells continues.

In other words, an embodiment of the method of the present invention may be a method for improving the culture performance of the animal cells. That is, an embodiment of the method of the present invention may be, for example, a method for improving the proliferation of the animal cells. An embodiment of the method of the present invention may also be, for example, a method for improving the survival rate of the animal cells. An embodiment of the method of the present invention may also be, for example, a method for improving production of the objective substance by the animal cells. An embodiment of the method of the present invention may also be, for example, a method for elongating the culture period of the animal cells. Furthermore, the present invention provides use of the active ingredient for improving the culture performance of the animal cells.

Furthermore, the active ingredient may function as an alternative to cysteine. The phrase "an active ingredient functions as an alternative to cysteine" may mean that a part or the whole of cysteine that can be used in the culture of the animal cells can be substituted with the active ingredient. Hence, by using the active ingredient, for example, the use amount of a cysteine-related substance such as cysteine in the culture of the animal cells may be reduced. By using the active ingredient, in particular, the use amount of a cysteine-related substance during a period where the animal cells are present in the culture medium at a sufficient viable cell density may be reduced. Whether the active ingredient functions as an alternative to cysteine can be confirmed by, for example, confirming that deterioration of the culture performance of the animal cells accompanying reduction in the use amount of cysteine can be partially or completely compensated by using the active ingredient. Whether the active ingredient functions as an alternative to cysteine can be confirmed by, specifically, for example, confirming that the culture performance of the animal cells is improved by culturing the animal cells with feeding of a feed medium containing the active ingredient according to the conditions described in Examples as compared to a case of culturing the animal cells with feeding of a feed medium containing none of the active ingredient and cysteine-related substances.

In other words, an embodiment of the method of the present invention may be a method for reducing the use amount of the cysteine-related substance in the culture of the animal cells. Furthermore, the present invention provides use of the active ingredient for reducing the use amount of the cysteine-related substance in the culture of the animal cells.

The term "culture of animal cells" is not limited to culture for the purpose of the proliferation of the animal cells, but may also include culture not for the purpose of the proliferation of the animal cells, such as culture for the purpose of maintenance of the animal cells and production of the objective substance by the animal cells.

The culture medium composition and culture conditions are not particularly limited, except that the culture is carried out in the presence of the active ingredient, so long as the purpose for the culture of the animal cells can be attained. For example, when the culture is carried out for the purpose of the proliferation of the animal cells, the culture medium composition and culture conditions can be configured so that the animal cells proliferate. Furthermore, for example, when the culture is carried out for the purpose of maintenance of the animal cells, the culture medium composition and culture conditions can be configured so that the animal cells are maintained (i.e., the animal cells survive). Furthermore, for example, when the culture is carried out for the purpose of production of the objective substance such as the objective protein by the animal cells, the culture medium composition and culture conditions can be configured so that the objective substance is produced (e.g., the objective protein is expressed). When the culture is carried out not for the purpose of the proliferation of the animal cells, the animal cells may or may not proliferate during the culture. Even when the culture is carried out not for the purpose of the proliferation of the animal cells, the animal cells may typically proliferate during the culture. The culture medium composition and culture conditions can be set according to various conditions such as the type of the animal cells. The culture can be carried out by, for example, using a usual culture medium and usual conditions used for culture of animal cells as they are, or after modifying them as required, except that the culture is carried out in the presence of the active ingredient.

The culture can be carried out by, for example, using a liquid culture medium. The culture medium to be used for the culture may be a commercially available culture medium, or may be a culture medium prepared by yourself. Specific examples of the culture medium usable for culture of animal cells include D-MEM (Dulbecco's Modified Eagle Medium), CELLiST Basal Media BASAL3, BASAL4P, and BASAL10 (Ajinomoto), Opti-MEM (Thermo Fisher Scientific), RPMI 1640 (Thermo Fisher Scientific), CD293 (Thermo Fisher Scientific), CHO-S-SFMII (Thermo Fisher Scientific), CHO-SF (Sigma-Aldrich), EX-CELL CD CHO (Sigma-Aldrich), EX-CELL^{™}302 (Sigma-Aldrich), IS CHO-CD (Irvine Scientific), IS CHO-CDXP (Irvine Scientific), and so forth. The culture medium may contain various culture medium components such as carbon sources, amino acids, vitamins, inorganic components, cysteine-related substances, pH buffers, growth factors, serum, serum albumin, selective agents, and gene expression inducers. Examples of the carbon sources glucose. Examples of the amino acids include 20 amino acids constituting proteins and derivatives thereof. The amino acids each may be, for example, L-isomer. The term "cysteine-related substance" collectively refers to cysteine and derivatives thereof, except for the active ingredient. Examples of the cysteine derivatives include cystine and thiazolidine derivatives. Examples of the thiazolidine derivatives include cysteinyl pyruvic acid. Particular examples of the cysteine-related substance include cysteine and cysteinyl pyruvic acid. Cysteine may be, for example, L-cysteine. Cysteine constituting the cysteine derivatives may be, for example, L-cysteine. Examples of the vitamins include vitamin A, vitamin B1, vitamin B2, vitamin B3, vitamin B5, vitamin B6, vitamin B7, vitamin B9, vitamin B12, vitamin C, vitamin D, vitamin E, and vitamin K, and precursors thereof. Examples of the inorganic components include sodium, potassium, calcium, magnesium, and phosphorus, and ions thereof, or trace elements such as Co, Cu, F, Fe, Mn, Mo, Ni, Se, Si, Ni, Bi, V, and Zn. Examples of the pH buffers include sodium bicarbonate, phosphate, and HEPES. Examples of the growth factors include insulin, IGF-1, and FGF.

Components that can form a salt each may be used as a free compound, a salt thereof, or a combination of them. That is, for example, the term "L-cysteine" may mean L-cysteine in the form of free compound, a salt thereof, or a combination of them, unless otherwise stated. For the salt, the descriptions concerning the salt of the active ingredient can be similarly applied. Furthermore, these components (e.g., free compounds or salts) each may include both non-hydrate and hydrate, unless otherwise stated.

The inoculation amount of the animal cells at the start of the culture, for example, may be 1×10³ cells/mL or more, 1×10⁴ cells/mL or more, 1×10⁵ cells/mL or more, 1×10⁶ cells/mL or more, or 1×10⁷ cells/mL, may be 1×10⁸ cells/mL or less, 1×10⁷ cells/mL or less, 1×10⁶ cells/mL or less, 1×10⁵ cells/mL or less, or 1×10⁴ cells/mL or less or may be within a range defined as a non-contradictory combination thereof.

The culture may be started, for example, under conditions where the animal cells are present in the culture medium at a sufficient viable cell density, or under conditions where the animal cells are not present in the culture medium at a sufficient viable cell density.

The phrase "animal cells are present in a culture medium at a sufficient viable cell density" may mean, for example, that the viable cell density of the animal cells in the culture medium is 1×10⁶ cells/mL or more, 2×10⁶ cells/mL or more, 3×10⁶ cells/mL or more, 4×10⁶ cells/mL or more, 5×10⁶ cells/mL or more, 6×10⁶ cells/mL or more, 7×10⁶ cells/mL or more, 8×10⁶ cells/mL or more, 9×10⁶ cells/mL or more, or 1×10⁷ cells/mL or more. The phrase "animal cells are present in a culture medium at a sufficient viable cell density" may also mean, in particular, that the viable cell density of the animal cells in the culture medium is 5×10⁶ cells/mL or more. Reaching of the animal cells to the sufficient viable cell density upon the culture is also referred to as "animal cells sufficiently proliferate".

The phrase "animal cells are not present in a culture medium at a sufficient viable cell density" may mean, for example, that the viable cell density of the animal cells in the culture medium is less than 1×10⁷ cells/mL, less than 9×10⁶ cells/mL, less than 8×10⁶ cells/mL, less than 7×10⁶ cells/mL, less than 6×10⁶ cells/mL, less than 5×10⁶ cells/mL, less than 4×10⁶ cells/mL, less than 3×10⁶ cells/mL, less than 2×10⁶ cells/mL, or less than 1×10⁶ cells/mL. The phrase "animal cells are not present in a culture medium at a sufficient viable cell density" may also mean, in particular, that the viable cell density of the animal cells in the culture medium is less than 5×10⁶ cells/mL.

The viable cell count can be measured by, for example, using a viable and dead cells auto-analyzer Vi-CELL^{™} XR (Beckman Coulter).

The culture may also be carried out separately as seed culture and main culture. When the culture is carried out separately as seed culture and main culture, it is sufficient that at least the main culture is carried out in the presence of the active ingredient. When the culture is carried out separately as seed culture and main culture, the seed culture may or may not be carried out in the presence of the active ingredient. When the culture is carried out separately as seed culture and main culture, terms regarding the culture, such as "culture period (period of culture)" and "start of culture", can be read as those regarding the main culture, unless otherwise stated. The culture conditions of the seed culture and the culture conditions of the main culture may or may not be the same. The culture conditions of the main culture can also be applied similarly to the culture conditions of the seed culture. When the culture is carried out for the purpose of production of the objective substance such as the objective protein, it is sufficient that the objective substance is produced at least during the main culture. For example, the animal cells may be allowed to sufficiently proliferate by seed culture, and then the objective substance such as the objective protein may be produced.

The culture can be carried out as batch culture, fed-batch culture, continuous culture, or a combination of these. Examples of the continuous culture include perfusion culture. The culture medium used at the start of the culture is also referred to as "starting medium" or "basal medium". The culture medium supplied to the culture system (e.g., starting medium) in the fed-batch culture or the continuous culture is also referred to as "feed medium". To supply a feed medium to the culture system in the fed-batch culture or the continuous culture is also referred to as "feed". The feeding may be carried out over the whole period of the culture, or may be carried out during only a partial period of the culture. The feeding may be carried out continuously, or may be carried out intermittently. Upon the culture (in particular, continuous culture such as perfusion culture), discharge of the culture broth may be carried out. The discharge of the culture broth may be carried out over the whole period of the culture, or may be carried out during only a partial period of the culture. The discharge of the culture broth may be carried out continuously, or may be carried out intermittently. The discharge of the culture broth and the feeding may or may not be carried out simultaneously. When the culture is carried out separately as seed culture and main culture, the culture schemes of the seed culture and the main culture may or may not be the same.

The various components such as the active ingredient may be contained in the starting medium, the feed medium, or both of them. That is, the various components such as the active ingredient may be supplied to the culture medium independently or in any combination during the culture. These components may be supplied once or a plurality of times, or may be continuously supplied. The compositions (e.g., the types and/or concentrations of contained components) of the starting medium and the feed medium may or may not be the same. That is, the types of the components contained in the starting medium may or may not be the same as those of the components contained in the feed medium. Furthermore, the concentrations of the components contained in the starting medium may or may not be the same as the concentrations of the components contained in the feed medium. The compositions of the starting medium and the feed medium may be the same, for example, when the feed medium is used for perfusion culture. Furthermore, two or more kinds of feed media having different compositions (e.g., containing components of different types and/or different concentrations) may be used. For example, when the feeding is intermittently carried out two or more times, the composition of the feed medium may or may not be the same for each feeding. Furthermore, the various components such as the active ingredient may be supplied to the culture medium in the form not contained in the feed medium, e.g., in the form of powder.

The culture may be carried out, for example, under a CO₂-containing atmosphere, such as 5 to 15% CO₂. The pH of the culture medium may be, for example, around neutral. The term "around neutral" may refer to, for example, pH 6 to 8, pH 6.5 to 7.5, or pH 6.8 to 7.2. The pH of the culture medium can be adjusted during the culture as required. The pH of the culture medium can be adjusted by using various alkaline or acidic substances such as ammonia gas, aqueous ammonia, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 30 to 38°C. The culture period, for example, may be 0.5 day or longer, 1 day or longer, 2 days or longer, 3 days or longer, 4 days or longer, 5 days or longer, 6 days or longer, 7 days or longer, 8 days or longer, 9 days or longer, 10 days or longer, 12 days or longer, 15 days or longer, or 20 days or longer, may be 60 days or shorter, 50 days or shorter, 40 days or shorter, 30 days or shorter, 25 days or shorter, 20 days or shorter, 15 days or shorter, 12 days or shorter, 10 days or shorter, 9 days or shorter, 8 days or shorter, or 7 days or shorter, or may be within a range defined as a non-contradictory combination thereof. The culture period may be, specifically, for example, 1 to 60 days, 3 to 25 days, or 5 to 20 days. Expression of a gene such as the objective protein gene may be induced as required upon the culture.

The phrase "culture is carried out in the presence of an active ingredient" may mean, for example, that the culture medium contains the active ingredient, i.e., the culture is carried out in the culture medium containing the active ingredient.

The concentration of the active ingredient in the culture medium, for example, may be 0.5 mM or more, 0.6 mM or more, 0.7 mM or more, 0.8 mM or more, 0.9 mM or more, 0.92 mM or more, 1 mM or more, 1.2 mM or more, 1.5 mM or more, 2 mM or more, 2.5 mM or more, 3 mM or more, 3.5 mM or more, 4 mM or more, 4.5 mM or more, 5 mM or more, 5.5 mM or more, 6 mM or more, 6.5 mM or more, 7 mM or more, 7.5 mM or more, 8 mM or more, 8.5 mM or more, 9 mM or more, 9.5 mM or more, or 10 mM or more, may be 20 mM or less, 15 mM or less, 12 mM or less, 10 mM or less, 9.5 mM or less, 9 mM or less, 8.5 mM or less, 8 mM or less, 7.5 mM or less, 7 mM or less, 6.5 mM or less, 6 mM or less, 5.5 mM or less, 5 mM or less, 4.5 mM or less, 4 mM or less, 3.5 mM or less, 3 mM or less, 2.5 mM or less, 2 mM or less, 1.5 mM or less, or 1 mM or less, or may be within a range defined as a non-contradictory combination thereof. The concentration of the active ingredient in the culture medium may be, in particular, 0.92 mM or more. The concentration of the active ingredient in the culture medium may be, specifically, for example, 0.5 to 20 mM, 1 to 15 mM, or 1.5 to 10 mM

The active ingredient may be contained in the culture medium over the whole period of the culture, or may be contained in the culture medium during only a partial period of the culture. That is, it is sufficient that the phrase "culture is carried out in the presence of an active ingredient" or "culture is carried out in a culture medium containing an active ingredient" means that the active ingredient is contained in the culture medium during at least a partial period of the culture, and the phrase does not necessary mean that the active ingredient is contained in the culture medium over the whole period of the culture. For example, the active ingredient may be contained in the culture medium at the start of the culture, or may be supplied to the culture medium after the start of the culture. Also, for example, the active ingredient may be contained in the culture medium at the start of the culture, and may be further supplied to the culture medium after the start of the culture (e.g., after consumption of the active ingredient).

The active ingredient, for example, may be contained in the culture medium at the concentration exemplified above over the whole period of the culture, or may be contained in the culture medium at the concentration exemplified above during only a partial period of the culture. That is, it is sufficient that the phrase "culture is carried out in the presence of an active ingredient at a certain concentration" or the phrase "culture is carried out in a culture medium having a certain concentration of an active ingredient" means that the concentration of the active ingredient in the culture medium is within the range of the certain concentration during at least a partial period of the culture, and the phrase does not necessary mean that the concentration of the active ingredient in the culture medium is within the range of the certain concentration over the whole period of the culture. For example, the active ingredient may be contained in the culture medium at the concentration exemplified above at the start of the culture, or may be supplied to the culture medium to provide the concentration exemplified above after the start of the culture. Also, for example, the active ingredient may be contained in the culture medium at the concentration exemplified above at the start of the culture, and may be further supplied to the culture medium to provide the concentration exemplified above after the start of the culture (e.g., after consumption of the active ingredient).

The "partial period of the culture" referred to in use of the active ingredient is not particularly limited, so long as the desired effect by the active ingredient is obtained. The "partial period of the culture" referred to in use of the active ingredient can be set according to various conditions such as the type of the active ingredient, the type of the animal cells, the length of the culture period, and a desired production amount of the objective substance. The "partial period" referred to in use of the active ingredient may be, for example, a period having a length of 10% or longer, 20% or longer, 30% or longer, 40% or longer, 50% or longer, 60% or longer, 70% or longer, 80% or longer, 90% or longer, 95% or longer, 97% or longer, or 99% or longer of the whole period of the culture. The "partial period" referred to in use of the active ingredient may also be, for example, a period having a length of 0.5 day or longer, 1 day or longer, 2 days or longer, 3 days or longer, 4 days or longer, 5 days or longer, 6 days or longer, 7 days or longer, 8 days or longer, 9 days or longer, 10 days or longer, 12 days or longer, or 15 days or longer.

The "partial period of the culture" referred to in use of the active ingredient may comprise, for example, a period where the animal cells are present in the culture medium at a sufficient viable cell density, or a partial period thereof. The "partial period of the period where the animal cells are present in the culture medium at a sufficient viable cell density " may be, for example, a period having a length of 50% or longer, 60% or longer, 70% or longer, 80% or longer, 90% or longer, 95% or longer, 97% or longer, or 99% or longer of the period where the animal cells are present in the culture medium at a sufficient viable cell density.

The concentration of the active ingredient in the culture medium may also be set to be the concentration exemplified above, for example, in terms of an average value over a specific period of the culture. That is, the phrase "culture is carried out in the presence of an active ingredient at a certain concentration" or the phrase "culture is carried out in a culture medium having a certain concentration of an active ingredient" may also mean that the average value of the concentration of the active ingredient in the culture medium over the specific period of the culture is within the range of the certain concentration. The term "average value of the concentration of an active ingredient in a culture medium over a specific period of culture" is not particularly limited so long as the transition of the concentration of the active ingredient in the culture medium over the specific period of the culture can be recognized, and may refer to, for example, the average value of the concentrations of the active ingredient in the culture medium measured every 60 minutes, every 30 minutes, every 20 minutes, or every 10 minutes over the specific period of the culture.

Examples of the "specific period of the culture" referred to in use of the active ingredient include the whole period of the culture and the period where the animal cells are present in the culture medium at a sufficient viable cell density. Particular examples of the "specific period of the culture" referred to in use of the active ingredient include the period where the animal cells are present in the culture medium at a sufficient viable cell density.

The phrase "culture is carried out in the presence of an active ingredient" may also mean, for example, that the active ingredient is supplied to the culture medium.

The active ingredient may be supplied to the culture medium over the whole period of the culture, or may be supplied to the culture medium during only a partial period of the culture. The supply of the active ingredient to the culture medium, for example, may be started or carried out during the period where the animal cells are present in the culture medium at a sufficient viable cell density, or may be started or carried out during the period where the animal cells are not present in the culture medium at a sufficient viable cell density. The active ingredient may be supplied to the culture medium, for example, continuously or intermittently. The active ingredient may be supplied to the culture medium, for example, every day or every few days.

The supply amount of the active ingredient to the culture medium, for example, may be 0.1 mM or more, 0.15 mM or more, 0.2 mM or more, 0.25 mM or more, 0.3 mM or more, 0.35 mM or more, 0.4 mM or more, 0.45 mM or more, 0.5 mM or more, 0.55 mM or more, 0.6 mM or more, 0.65 mM or more, 0.7 mM or more, 0.75 mM or more, 0.8 mM or more, 0.85 mM or more, 0.9 mM or more, 0.95 mM or more, or 1 mM or more, may be 10 mM or less, 7 mM or less, 5 mM or less, 3 mM or less, 2 mM or less, 1.5 mM or less, 1.2 mM or less, 1 mM or less, 0.95 mM or less, 0.9 mM or less, 0.85 mM or less, 0.8 mM or less, 0.75 mM or less, 0.7 mM or less, 0.65 mM or less, 0.6 mM or less, 0.55 mM or less, 0.5 mM or less, 0.45 mM or less, 0.4 mM or less, 0.35 mM or less, or 0.3 mM or less, or may be within a range defined as a non-contradictory combination thereof, per day over the specific period of the culture. The supply amount of the active ingredient to the culture medium may be, specifically, for example, 0.1 to 10 mM, 0.1 to 2 mM, 0.2 to 1.5 mM, or 0.3 to 1 mM per day over the specific period of the culture.

When the active ingredient is supplied to the culture medium by feeding of the fed medium, the concentration of the active ingredient in the feed medium is not particularly limited, so long as a desired supply amount of the active ingredient is attained. The concentration of the active ingredient in the feed medium may be set to, for example, be within the range of the concentration of the active ingredient in the culture medium exemplified above. The concentration of the active ingredient in the feed medium may be set to be within the range of the concentration the active ingredient in the culture medium exemplified above, for example, when the feed medium is used for perfusion culture. The concentration of the active ingredient in the feed medium, for example, may be a concentration of 1 time or more, 1.1 times or more, 1.3 times or more, 1.5 times or more, 2 times or more, 3 times or more, 5 times or more, 7 times or more, 10 times or more, 15 times or more, or 20 times or more the concentration of the active ingredient in the culture medium exemplified above, may be a concentration of 100 times or less, 70 times or less, 50 times or less, 30 times or less, 20 times or less, 10 times or less, or 5 times or less the concentration of the active ingredient in the culture medium exemplified above, or may be within a range defined as a non-contradictory combination thereof. The concentration of the active ingredient in the feed medium, for example, may be 1 mM or more, 2 mM or more, 5 mM or more, 10 mM or more, 15 mM or more, 20 mM or more, 25 mM or more, 30 mM or more, 35 mM or more, 40 mM or more, 45 mM or more, 50 mM or more, 55 mM or more, or 60 mM or more, may be the saturated concentration or less, 100 mM or less, 95 mM or less, 90 mM or less, 85 mM or less, 80 mM or less, 75 mM or less, 70 mM or less, 65 mM or less, 60 mM or less, 55 mM or less, 50 mM or less, 45 mM or less, 40 mM or less, 35 mM or less, 30 mM or less, 25 mM or less, or 20 mM or less, or may be within a range defined as a non-contradictory combination thereof. The concentration of active ingredient in the feed medium may be, specifically, for example, 5 to 100 mM, 10 to 90 mM, or 20 to 80 mM.

The culture may also be carried out in the presence of the cysteine-related substance. That is, the culture of the animal cells may also be carried out by using the cysteine-related substance. The cysteine-related substance may be used for, for example, making the animal cells sufficiently proliferate. The cysteine-related substance may be used, specifically, for example, in cases of starting the culture under conditions where the animal cells are not present in the culture medium at a sufficient viable cell density.

The phrase "culture is carried out in the presence of a cysteine-related substance" may mean, for example, that the culture medium contains the cysteine-related substance, i.e., the culture is carried out in the culture medium containing the cysteine-related substance.

The concentration of the cysteine-related substance in the culture medium, for example, may be 0.1 mM or more, 0.2 mM or more, 0.3 mM or more, 0.4 mM or more, 0.5 mM or more, 0.6 mM or more, 0.7 mM or more, 0.8 mM or more, 0.9 mM or more, 1 mM or more, 1.1 mM or more, 1.2 mM or more, 1.3 mM or more, 1.4 mM or more, or 1.5 mM or more, may be 10 mM or less, 7 mM or less, 5 mM or less, 3 mM or less, 2.5 mM or less, 2 mM or less, 1.5 mM or less, 1.4 mM or less, 1.3 mM or less, 1.2 mM or less, 1.1 mM or less, 1 mM or less, 0.92 mM or less, 0.9 mM or less, 0.8 mM or less, 0.7 mM or less, 0.6 mM or less, or 0.5 mM or less, or may be within a range defined as a non-contradictory combination thereof, in terms of the concentration of cysteine. The term "concentration of a cysteine-related substance in terms of the concentration of cysteine" refers to a value obtained by multiplying the concentration of the cysteine-related substance by the number of moieties corresponding to cysteine within one molecule of the cysteine-related substance. For example, when the concentration of cystine is X, the concentration of cystine in terms of the concentration of cysteine is calculated to be 2X. The concentration of the cysteine-related substance in the culture medium may be, specifically, for example, 0.1 to 10 mM, 0.1 to 3 mM, 0.5 to 2 mM, or 1 to 1.5 mM in terms of the concentration of cysteine.

The cysteine-related substance may be contained in the culture medium over the whole period of the culture, or may be contained in the culture medium during only a partial period of the culture. That is, it is sufficient that the phrase "culture is carried out in the presence of a cysteine-related substance" or "culture is carried out in a culture medium containing a cysteine-related substance" means that the cysteine-related substance is contained in the culture medium during at least a partial period of the culture, and the phrase does not necessary mean that the cysteine-related substance is contained in the culture medium over the whole period of the culture. For example, the cysteine-related substance may be contained in the culture medium at the start of the culture, or may be supplied to the culture medium after the start of the culture. Also, for example, the cysteine-related substance may be contained in the culture medium at the start of the culture, and may be further supplied to the culture medium after the start of the culture (e.g., after consumption). The cysteine-related substance may be contained in the culture medium, in particular, at least at the start of the culture.

The cysteine-related substance, for example, may be contained in the culture medium at the concentration exemplified above over the whole period of the culture, or may be contained in the culture medium at the concentration exemplified above during only a partial period of the culture. That is, it is sufficient that the phrase "culture is carried out in the presence of a cysteine-related substance at a certain concentration" or the phrase "culture is carried out in a culture medium having a certain concentration of a cysteine-related substance" means that the concentration of the cysteine-related substance in the culture medium is within the range of the certain concentration during at least a partial period of the culture, and the phrase does not necessary mean that the concentration of the cysteine-related substance in the culture medium is within the range of the certain concentration over the whole period of the culture. For example, the cysteine-related substance may be contained in the culture medium at the concentration exemplified above at the start of the culture, or may be supplied to the culture medium to provide the concentration exemplified above after the start of the culture. Also, for example, the cysteine-related substance may be contained in the culture medium at the concentration exemplified above at the start of the culture, and may be further supplied to the culture medium to provide the concentration exemplified above after the start of the culture (e.g., after consumption). The cysteine-related substance may be contained in the culture medium at the concentration exemplified above, in particular, at least at the start of the culture.

The "partial period of the culture" referred to in use of the cysteine-related substance is not particularly limited, so long as the desired effect by the cysteine-related substance is obtained. The "partial period of the culture" referred to in use of the cysteine-related substance can be set according to various conditions such as the type of the animal cells, the length of the culture period, and a desired production amount of the objective substance. The "partial period of the culture " referred to in use of the cysteine-related substance, for example, may be a period having a length of 1% or more, 3% or more, 5% or more, 10% or more, 20% or more, 30% or more, 40% or more, or 50% or more of the whole period of the culture, may be a period having a length of 90% or less, 80% or less, 70% or less, 60% or less, 50% or less, 40% or less, 30% or less, 20% or less, or 10% or less of the whole period of the culture, or may be a period having a length defined as a non-contradictory combination thereof. The "partial period" referred to in use of the cysteine-related substance, for example, may be a period having a length of 0.5 day or longer, 1 day or longer, 2 days or longer, 3 days or longer, 4 days or longer, or 5 days or longer, may be a period having a length of 15 days or shorter, 10 days or shorter, 7 days or shorter, 5 days or shorter, or 3 days or shorter, or may be a period having a length defined as a non-contradictory combination thereof.

The "partial period of the culture" referred to in use of the cysteine-related substance may comprise, for example, a period where the animal cells are not present in the culture medium at a sufficient viable cell density, or a partial period thereof. The "partial period of the period where the animal cells are not present in the culture medium at a sufficient viable cell density " may be, for example, a period having a length of 50% or longer, 60% or longer, 70% or longer, 80% or longer, 90% or longer, 95% or longer, 97% or longer, or 99% or longer of the period where the animal cells are not present in the culture medium at a sufficient viable cell density.

The concentration of the cysteine-related substance in the culture medium may also be set to be the concentration exemplified above, for example, in terms of an average value over a specific period of the culture. That is, the phrase "culture is carried out in the presence of a cysteine-related substance at a certain concentration" or the phrase "culture is carried out in a culture medium having a certain concentration of a cysteine-related substance" may also mean that the average value of the concentration of the cysteine-related substance in the culture medium over the specific period of the culture is within the range of the certain concentration. The term "average value of the concentration of a cysteine-related substance in a culture medium over a specific period of culture" is not particularly limited so long as the transition of the concentration of the cysteine-related substance in the culture medium over the specific period of the culture can be recognized, and may refer to, for example, the average value of the concentrations of the cysteine-related substance in the culture medium measured every 60 minutes, every 30 minutes, every 20 minutes, or every 10 minutes over the specific period of the culture.

Examples of the "specific period of the culture" referred to in use of the cysteine-related substance include the whole period of the culture and the period where the animal cells are not present in the culture medium at a sufficient viable cell density. Particular examples of the "specific period of the culture" referred to in use of the cysteine-related substance include the period where the animal cells are not present in the culture medium at a sufficient viable cell density.

The phrase "culture is carried out in the presence of a cysteine-related substance" may also mean, for example, that the cysteine-related substance is supplied to the culture medium.

The cysteine-related substance may be supplied to the culture medium over the whole period of the culture, or may be supplied to the culture medium during only a partial period of the culture. The supply of the cysteine-related substance to the culture medium, for example, may be started or carried out during the period where the animal cells are present in the culture medium at a sufficient viable cell density, or may be started or carried out during the period where the animal cells are not present in the culture medium at a sufficient viable cell density. The cysteine-related substance may be supplied to the culture medium, for example, continuously or intermittently. The cysteine-related substance may be supplied to the culture medium, for example, every day or every few days.

The supply amount of the cysteine-related substance to the culture medium may be set, for example, so that such a concentration of the cysteine-related substance in the culture medium as exemplified above is attained.

The supply amount of the cysteine-related substance to the culture medium may be, for example, 50% or less, 40% or less, 30% or less, 20% or less, 10% or less, 5% or less, 2% or less, 1% or less, or zero in molar ratio to the supply amount of the active ingredient to the culture medium in terms of the concentration of cysteine over the period where the animal cells are present in the culture medium at a sufficient viable cell density.

The supply amount of the cysteine-related substance to the culture medium may be, for example, 1 mM or less, 0.5 mM or less, 0.2 mM or less, 0.1 mM or less, 0.01 mM or less, 0.001 mM or less, or zero in terms of the concentration of cysteine over the period where the animal cells are present in the culture medium at a sufficient viable cell density.

When the cysteine-related substance is supplied to the culture medium by feeding of the fed medium, the concentration of the cysteine-related substance in the feed medium is not particularly limited, so long as a desired supply amount of the cysteine-related substance is attained. The concentration of the cysteine-related substance in the feed medium may be set to, for example, be within the range of the concentration of the cysteine-related substance in the culture medium exemplified above. The concentration of the cysteine-related substance in the feed medium may be set to be within the range of the concentration the cysteine-related substance in the culture medium exemplified above, for example, when the feed medium is used for perfusion culture. The concentration of the cysteine-related substance in the feed medium, for example, may be a concentration of 1 time or more, 1.1 times or more, 1.3 times or more, 1.5 times or more, 2 times or more, 3 times or more, 5 times or more, 7 times or more, 10 times or more, 15 times or more, or 20 times or more the concentration of the cysteine-related substance in the culture medium exemplified above, may be a concentration of 100 times or less, 70 times or less, 50 times or less, 30 times or less, 20 times or less, 10 times or less, or 5 times or less the concentration of the cysteine-related substance in the culture medium exemplified above, or may be within a range defined as a non-contradictory combination thereof.

The concentrations of the various components can be measured by known methods used for detection or identification of compounds. Examples of such methods include, for example, HPLC, UPLC, LC/MS, GC/MS, and NMR These methods can also be used for confirming generation of the objective substance. One of these methods may be independently used, or two or more of these methods may be used in an appropriate combination.

The animal cells can be cultured as described above, when the animal cells have an objective substance-producing ability such as an objective protein-producing ability, by culturing the animal cells as described above, the objective substance is generated (e.g., the objective protein is expressed) and thereby a culture broth containing the objective substance is obtained. The objective substance such as the objective protein may be accumulated, specifically, in a culture medium, on a cell surface layer, in cells, or in/on a combination thereof.

Hereinafter, operations such as confirmation of generation of the objective protein, collection of the objective protein, and purification of the objective protein will be explained with reference to cases of producing the objective protein. Such operations can be carried out for the objective substance other than the objective protein as required.

Generation of the objective protein can be confirmed by known methods used for detection or identification of proteins. Examples of such methods include, for example, SDS-PAGE, Western blotting, mass spectrometry, N-terminal amino acid sequence analysis, and enzyme activity measurement. One of these methods may be independently used, or two or more of these methods may be used in an appropriate combination.

The objective protein can be collected as required. Specifically, the objective protein can be collected as an appropriate fraction containing the objective protein. Examples of such a fraction include, for example, a culture broth, a culture supernatant, cultured cells, and a processed product of cultured cells (a disruption product, a lysate, or an extract (cell-free extract)). The cultured cells may also be obtained, for example, in the form of immobilized cells immobilized on a carrier such as acrylamide and carrageenan.

The objective protein may further be purified to a desired extent.

When the objective protein is accumulated in the culture medium, for example, solids such as cells can be removed from the culture broth by centrifugation or the like, and then the objective protein can be purified from the culture supernatant.

When the objective protein is accumulated in cells, for example, the cells can be subject to a treatment such as disruption, lysis, or extraction, and then the objective protein can be purified from the treated product. The cells can be collected from the culture broth by centrifugation or the like. The treatment such as disruption, lysis, or extraction of cells can be carried out by known methods. Examples of such methods include, for example, disruption by ultrasonication, disruption in Dyno-Mill, disruption in bead mill, disruption with French press, and lysozyme treatment. One of these methods may be used alone, or two or more of these methods may be used in combination as required.

When the objective protein is accumulated on a cell surface layer, for example, the objective protein can be solubilized, and then the objective protein can be purified from the solubilized product. The solubilization can be carried out by known methods. Examples of such methods include, for example, an increase in a salt concentration and use of a surfactant. One of these methods may be independently used, or two or more of these methods may be used in an appropriate combination.

Purification of the objective protein, such as purification of the objective protein from such a supernatant, treated product, or solubilized product as described above, can be carried out by known methods used for purification of proteins. Examples of such methods include, for example, ammonium sulfate fractionation, ion exchange chromatography, hydrophobic chromatography, affinity chromatography, gel filtration chromatography, and isoelectric precipitation. One of these methods may be independently used, or two or more of these methods may be used in an appropriate combination.

The objective protein may be obtained in the form of a free enzyme, or may be obtained in the form of an immobilized enzyme immobilized on a solid phase such as a resin.

The collected objective protein may be made into a formulation as required. The dosage form of the formulation is not particularly limited, and can be appropriately chosen according to various conditions such as use purpose of the objective protein. Examples of the dosage form include, for example, solution, suspension, powder, tablet, pill, and capsule. For preparing such a formulation, for example, pharmaceutically acceptable additives such as excipients, binders, disintegrating agents, lubricants, stabilizers, corrigents, odor-masking agents, perfumes, diluents, and surfactants can be used.

### <3> Culture medium of the present invention

The culture medium of the present invention is a culture medium for culturing animal cells, which culture medium contains the active ingredient. The culture medium of the present invention can be used for, for example, the method of the present invention.

For the culture medium of the present invention, for example, the aforementioned descriptions concerning the culture medium (including the descriptions concerning the feed medium) to be used for the method of the present invention can be similarly applied.

The culture medium of the present invention may or may not further contain the cysteine-related substance.

The culture medium of the present invention may be, for example, a basal medium (starting medium) or a feed medium. The feed medium may be, for example, a feed medium to be used for fed-batch culture or continuous culture. The feed medium may be, for example, in particular, a feed medium to be used for perfusion culture.

For the concentration of the active ingredient in the culture medium of the present invention, for example, the aforementioned descriptions concerning the concentration of the active ingredient in the culture medium (including the descriptions concerning the concentration of the active ingredient in the feed medium) in the method of the present invention can be similarly applied.

For the concentration of the cysteine-related substance in the culture medium of the present invention, for example, the aforementioned descriptions concerning the concentration of the cysteine-related substance in the culture medium (including the descriptions concerning the concentration of the cysteine-related substance in the feed medium) in the method of the present invention can be similarly applied.

### Examples

Hereinafter, the present invention will be more specifically explained with reference to non-limiting examples.

L-cysteine used in the following experiments was L-cysteine hydrochloride monohydrate. All L-cysteine derivatives used in the following experiments were free compounds, unless otherwise stated.

### Example 1: Evaluation of effect of addition of L-cysteine derivatives to basal medium on proliferation of CHO cells

In this Example, an effect of addition of L-cysteine derivatives to a basal medium on proliferation of CHO cells was evaluated. As the L-cysteine derivatives, (2RS,4R)-2-methyl-2,4-thiazolidinedicarboxylic acid 2-ethyl ester (hereinafter, also referred to as "Compound 1") and (2RS,4R)-2-methyl-2,4-thiazolidinedicarboxylic acid (hereinafter, also referred to as "Compound 2") were used. As the CHO cells, CHO cells modified to produce a monoclonal antibody were used.

### (1) Preparation of basal medium

CELLiST (registered trademark) Basal media (Ajinomoto Co., Ltd.: BASAL3) without L-cysteine was prepared, to obtain a culture medium without addition of L-cysteine. The culture medium without addition of L-cysteine was added with L-cysteine, Compound 1, or Compound 2, to prepare a culture medium containing 1.3 mM L-cysteine, 1.3 mM Compound 1, or 1.3 mM Compound 2. Each culture medium was added with LR3-IGF-1 (SIGMA-ALDRICH: I1271-1MG, final concentration of 50 µg/mL) and L-Glutamine (Thermo Fisher SCIENTIFIC: 25030081, final concentration of 6 mM), to prepare a basal medium.

### (2) Culture experiment

A 3 mL-aliquot of a CHO cell suspension adjusted to 3×10⁵ cells/mL using the prepared basal medium was seeded into a 6 well plate (CORNING: 3471), and cultured for 11 days. The culture was carried out with n=3 for all experimental groups. The culture temperature was 37°C and the agitation speed was 110 rpm. A cell culture broth was sampled on days 7 and 11 from the start of the culture, and the viable cell count was measured using a viable/dead cells autoanalyzer Vi-CELL^{™} XR (Beckman Coulter).

### (3) Culture results

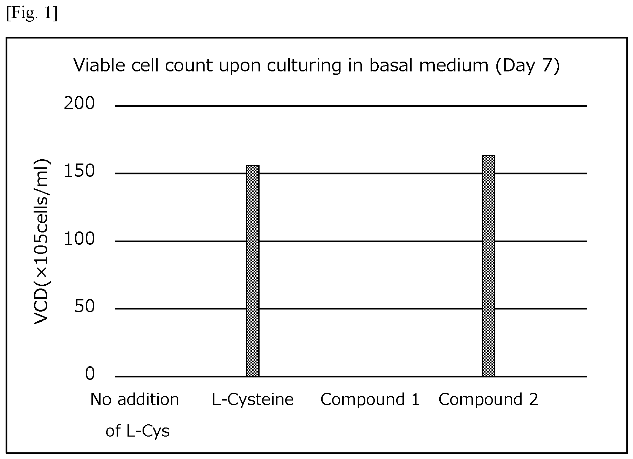

The viable cell count on day 7 of culture is shown in Fig. 1. Proliferation of cells was observed for the experimental group using L-cysteine, whereas no proliferation of cells was observed for the experimental group without addition of L-cysteine. Furthermore, no proliferation of cells was observed for the experimental group using Compound 1 as with the experimental group without addition of L-cysteine. That is, it was unable to substitute the whole of L-cysteine in the basal medium with Compound 1. By contrast, proliferation of cells observed for the experimental group using Compound 2 was almost equivalent to that of the experimental group using L-cysteine. That is, Compound 2 was able to be used as an alternative for L-cysteine.

### Example 2: Evaluation of effect of addition of L-cysteine derivatives to feed medium on proliferation of CHO cells and antibody production (1)

In this Example, an effect of addition of L-cysteine derivatives to a feed medium on proliferation of CHO cells and antibody production was evaluated. As the L-cysteine derivatives, Compounds 1 and 2 were used. As the CHO cells, CHO cells modified to produce a monoclonal antibody were used.

### (1) Preparation of basal medium

CELLiST (registered trademark) Basal media (Ajinomoto Co., Ltd.: BASAL3; containing 1.3 mM L-cysteine) was added with LR3-IGF-1 (SIGMA-ALDRICH: I1271-1MG, final concentration of 50 µg/mL) and L-Glutamine (Thermo Fisher SCIENTIFIC: 25030081, final concentration of 6 mM), to prepare a basal medium.

### (2) Preparation of feed medium

Feed media (9 kinds in total) each containing 82 g/L Cellvento (registered trademark) Feed-210 (Merck: 1.02488.0005), 70 g/L glucose (Wako Pure Chemical Industries, Ltd.: 043-31163), and L-cysteine, Compound 1, or Compound 2 each at 14.5 mM, 29 mM, or 58 mM were prepared. In addition, a feed medium without addition of L-cysteine, which only contained 82 g/L Cellvento (registered trademark) Feed-210 and 70 g/L glucose, was prepared. Separately, a tyrosine concentrate (L-tyrosine disodium salt dihydrate (Merck: 122666-87-9) 149.64 g/L, pH 11.3) was prepared.

### (3) Culture experiment

A 30 mL-aliquot of a CHO cell suspension adjusted to 3×10⁵ cells/mL using the prepared basal medium was seeded into 125 mL-volume flasks (CORNING: 431143), and cultured for 14 days. The culture was carried out with n=2 for all experimental groups. The culture temperature was 37°C and the agitation speed was 110 rpm. A 1.8 mL-aliquot of each of the prepared feed media and a 60 µL-aliquot of the tyrosine concentrate were added on days 4, 7, 9, and 11 from the start of the culture. A cell culture broth was sampled on days 4, 7, 9, 11, and 14 from the start of the culture, and the viable cell count was measured using a viable/dead cells autoanalyzer Vi-CELL^{™} XR (Beckman Coulter), and the antibody production amount was measured using Octet QK (FORTEBIO).

### (4) Culture results

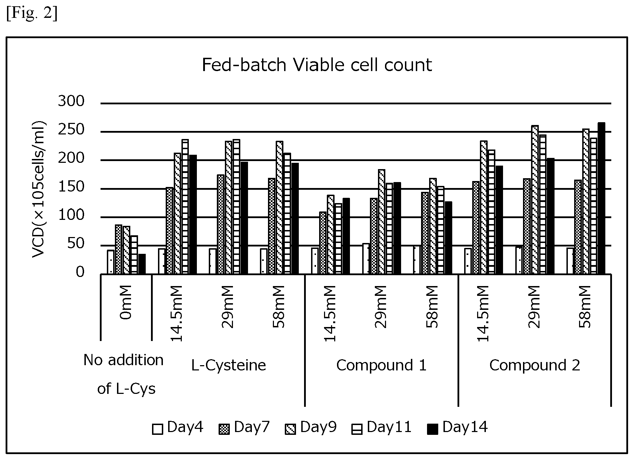

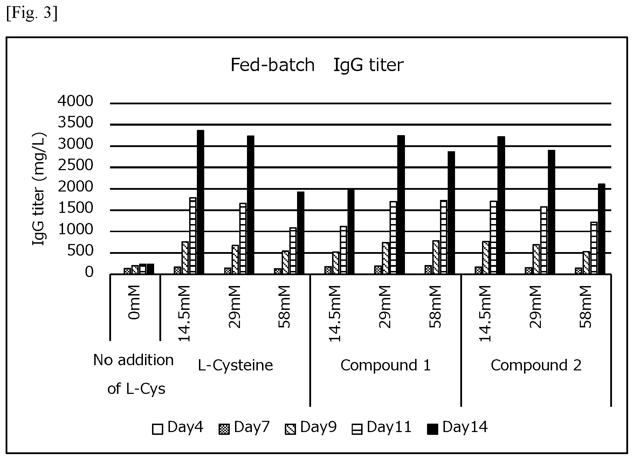

The results are shown in Figs. 2 and 3. The viable cell count and the antibody production amount of the experimental group without addition of L-cysteine were lower than those of the other experimental groups. By contrast, the antibody production amount observed for the experimental groups using Compound 1 and Compound 2 was almost equivalent to that of the experimental group of L-cysteine. That is, Compound 1 was able to be used as an alternative for L-cysteine in the feed medium, whereas it was unable to be used as an alternative for L-cysteine in the basal medium. This is presumed to be due to that the viable cell count reached 40×10⁵ cells/mL or higher on day 4 from the start of the culture, and thereby the ester degradation activity of cells sufficiently increased.

### Example 3: Evaluation of stability of L-cysteine derivatives in feed medium

In this Example, the stability of L-cysteine derivatives in a feed medium was evaluated. As the L-cysteine derivatives, Compounds 1 and 2 were used.

A feed medium containing 82 g/L Cellvento (registered trademark) Feed-210 (Merck: 1.02488.0005), 70 g/L glucose (Wako Pure Chemical Industries, Ltd.: 043-31163), and 58 mM L-cysteine, 58 mM Compound 1, or 58 mM Compound 2, and a feed medium without addition of L-cysteine, which only contained 82 g/L Cellvento (registered trademark) Feed-210 and 70 g/L glucose were prepared at pH 5.2 to 5.6, which are described in the instruction of Feed-210, and at pH 7.0, which is a neutral pH condition used for usual cultivation, to obtain 8 kinds of feed media in total. All of the feed media were prepared as transparent solutions.

After each of the feed media was stored at 5°C in the dark, crystal precipitation was confirmed by visual observation. The results are shown in Table 1. In the table, "-" indicates that no crystal precipitation was observed. In the table, "+" to "++++" indicate that crystal precipitation was observed, and the higher the number of "+", the greater the degree of precipitation. Under the addition condition, significant precipitation was observed from one week after the start of storage. Under the condition of adding Compound 2, precipitation was observed from 22 days after the start of storage. By contrast, under the condition without addition of L-cysteine and under the condition of adding Compound 1, the solutions remained clear until 30 days after the start of storage, and no crystal precipitation was observed. Hence, it was revealed that Compound 1 is more stable than Compound 2 in the culture medium.

**Table 1. Stability of compounds in feed medium**

| Compounds added to Feed media | Feed media pH | Days | | | | |
|---|---|---|---|---|---|---|
| | | 0 | 1 | 7 | 22 | 30 |
| No addition of L-Cysteine | 5.2 to 5.6 | - | - | - | - | - |
| 58 mM L-Cysteine hydrochloride monohydrate | | - | - | +++ | +++ | +++ |
| 58 mM Compound 1 | | - | - | - | - | - |
| 58 mM Compound 2 | | - | - | - | + | +++ |
| No addition of L-Cysteine | 7 | - | - | - | - | - |
| 58 mM L-Cysteine hydrochloride monohydrate | | - | - | +++ | +++ | +++ |
| 58 mM Compound 1 | | - | - | - | - | - |
| 58 mM Compound 2 | | - | - | - | ++ | +++ |

### Example 4: Production of L-cysteine derivatives

### (Production Example 1)

(2RS,4R)-2-methyl-2,4-thiazolidinedicarboxylic acid 2-methyl ester (hereinafter referred to as "Compound 3") mainly containing the 2S compound thereof was obtained by the following method. The structure of the 2S compound of Compound 3 is shown in the following formula (I-3S).

L-Cysteine (2.95 g, 24.52 mmol) was suspended in water (10 mL), and methyl pyruvate (4 mL, 44.27 mmol) was added thereto dropwise. The reaction mixture was stirred at room temperature for 18 hours, and then precipitated crystals were separated by filtration. The filtrate was left at room temperature, and a solid that further precipitated was separated by filtration, to obtain needle-like crystals mainly containing the 2S compound of Compound 3 (approximately 95% de). The needle-like crystals mainly containing the 2S compound are hereinafter also referred to as "Compound 3S".

### <Analysis data>

Yield 83 %; ESI MS m/z 206.1 (M+H)⁺; ¹H NMR (400 MHz, CD₃OD) δ 4.34 (1H, dd, J=6.8, 8.4 Hz), 3.74 (3H, s), 3.38 (1H, dd, J=6.8, 10.0 Hz), 3.13 (1H, dd, J=8.4, 10.0 Hz), 1.84 (3H, s).

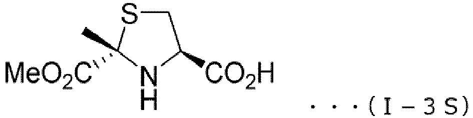

### (Production Example 2)

(2RS,4R)-2-methyl-2,4-thiazolidinedicarboxylic acid 2-methyl ester (Compound 3) mainly containing the 2R compound thereof was obtained by the following method. The structure of the 2R compound of Compound 3 is shown in the following formula (I-3R).

L-Cysteine (2.95 g, 24.35 mmol) was suspended in 50% methanol aqueous solution (40 mL), and methyl pyruvate (4 mL, 44.27 mmol) was added thereto dropwise. The reaction mixture was stirred at room temperature for 3 hours, and then methanol was removed under reduced pressure. The reaction mixture was added with ethyl acetate and subject to partitioning extraction. The organic layer was dried by dehydration with anhydrous sodium sulfate, and then concentrated and dried under reduced pressure. The residue was dissolved in ethyl acetate, heptane was added thereto, and the mixture was left to stand at -20°C. The precipitated solid was separated by filtration, to obtain a mixture of the 2S and 2R compounds of Compound 3 as a white solid. Separately, the mother liquor was concentrated and dried under reduced pressure, to obtain a white solid mainly containing the 2R" compound of Compound 3 (approximately 80% de). The white solid mainly containing the 2R compound are hereinafter also referred to as "Compound 3R".

### <Analysis data>

Yield 25 %; ESI MS m/z 206.1 (M+H)⁺; ¹H NMR (400 MHz, CD₃OD) δ 4.10 (1H, dd, J=6.0, 10.4 Hz), 3.82 (3H, s), 3.45 (1H, dd, J=6.0, 10.4 Hz), 2.93 (1H, t, J=10.4 Hz), 1.70 (3H, s).

Separately, it was confirmed that a sample containing a little more 2R compound of Compound 3 (approximately 60% de) is obtained by quickly subjecting a supernatant obtained by suspending an ethyl acetate extract obtained by the same method as above to partitioning extraction using ethyl acetate.

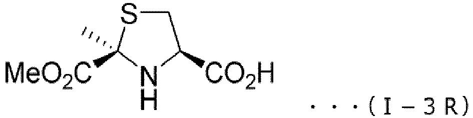

### (Production Example 3)

(2RS,4R)-2-methyl-2,4-thiazolidinedicarboxylic acid 2-ethyl ester (Compound 1) was obtained by the following method. The structure of the 2S compound of Compound 1 is shown in the following formula (I- 1S) and the structure of the 2R compound of Compound 1 is shown in the following formula (I-1R).

Compound 1 (diastereomeric mixture) was obtained as a white solid using L-cysteine and ethyl pyruvate as raw materials by the same method as in Production Example 1.

### <Analysis data>

Yield 82 %; 2S compound: ESI MS m/z 220.1 (M+H)⁺; ¹H NMR (400 MHz, CD₃OD) δ 4.34 (1H, dd, J=6.8, 8.4 Hz), 4.20 (2H, m), 3.38 (1H, dd, J=6.8, 10.4 Hz), 3.14 (1H, dd, J=8.4, 10.4 Hz), 1.84 (3H, s), 1.32 (3H, t, J=7.2 Hz); 2R compound: ESI MS m/z 220.1 (M+H)⁺; ¹H NMR (400 MHz, CD₃OD) δ 4.27 (2H, m), 4.11 (1H, dd, J=6.0, 10.0 Hz), 3.46 (1H, dd, J=6.0, 10.0 Hz), 2.94 (1H, t, J=10.0 Hz), 1.70 (3H, s), 1.29 (3H, t, J=7.2 Hz).

Separately, L-cysteine hydrochloride monohydrate (4.98 g, 28.35 mmol) was suspended in water (29.5 mL), 8% sodium hydroxide aqueous solution (16.84 mL) was added thereto little by little to adjust the pH to 6.99, and then ethyl pyruvate (3.8 mL, 34.12 mmol) was added thereto dropwise. The reaction mixture was stirred at room temperature for 10 hours and then at 10°C for 12 hours, and precipitated crystals were separated by filtration, to obtain needle-like crystals mainly containing the 2R compound of Compound 1 (approximately 90% de).

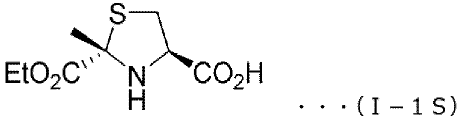

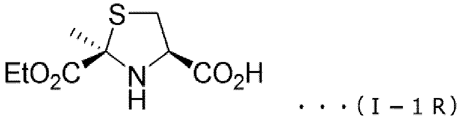

### (Production Example 4)

(2RS,4R)-2-methyl-2,4-thiazolidinedicarboxylic acid 2-i-propyl ester (hereinafter referred to as "Compound 4") was obtained by the following method. The structures of the 2S and 2R compounds of Compound 4 are shown in the following formulas (I-4S) and (I-4R), respectively.

Compound 4 (diastereomeric mixture) was obtained as a white solid using L-cysteine and i-propyl pyruvate as raw materials by the same method as in Production Example 1.

### <Analysis data>

Yield 51 %; 2S compound: ESI MS m/z 233.9 (M+H)⁺; ¹H NMR (400 MHz, CD₃OD) δ 5.00 (1H, m), 4.34 (1H, dd, J=6.8, 8.0 Hz), 3.38 (1H, dd, J=6.8, 10.4 Hz), 3.13 (1H, dd, J=8.0, 10.4 Hz), 1.82 (3H, s), 1.28 (3H, d, J=6.0 Hz), 1.27 (3H, d, J=6.4 Hz); 2R compound: ESI MS m/z 233.9 (M+H)⁺; ¹H NMR (400 MHz, CD₃OD) δ 5.08 (1H, m), 4.10 (1H, dd, J=6.0, 10.4 Hz), 3.45 (1H, dd, J=6.0, 10.4 Hz), 2.93 (1H, t, J=10.4 Hz), 1.69 (3H, s), 1.31 (3H, d, J=6.4 Hz), 1.30 (3H, d, J=6.4 Hz).

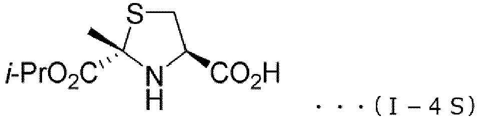

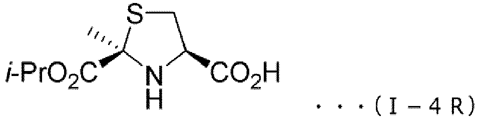

### (Production Example 5)

(2RS,4R)-2-methyl-2,4-thiazolidinedicarboxylic acid 2-n-butyl ester (hereinafter referred to as "Compound 5") was obtained by the following method. The structures of the 2S and 2R compounds of Compound 5 are shown in the following formulas (I-5S) and (I-5R), respectively.

Compound 5 (diastereomeric mixture) was obtained as a white solid using L-cysteine and n-butyl pyruvate as raw materials by the same method as in Production Example 1.

### <Analysis data>

Yield 41 %; 2S compound: ESI MS m/z 247.9 (M+H)⁺; ¹H NMR (400 MHz, CD₃OD) δ 4.34 (1H, dd, J=6.8, 8.0 Hz), 4.15 (2H, t, J=6.4 Hz), 3.38 (1H, dd, J=6.8, 10.4 Hz), 3.14 (1H, dd, J=8.0, 10.4 Hz), 1.84 (3H, s), 1.69-1.63 (2H, m), 1.50-1.40 (2H, m), 0.98 (3H, t, J=7.2 Hz); 2R compound: ESI MS m/z 247.9 (M+H)⁺; ¹H NMR (400 MHz, CD₃OD) δ 4.29-4.18 (2H, m), 4.10 (1H, dd, J=6.0, 10.4 Hz), 3.46 (1H, dd, J=6.0, 10.4 Hz), 2.93 (1H, t, J=10.4 Hz), 1.70 (3H, s), 1.69-1.63 (2H, m), 1.50-1.40 (2H, m), 0.99 (3H, t, J=7.6 Hz).

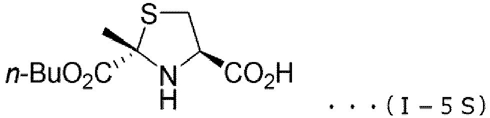

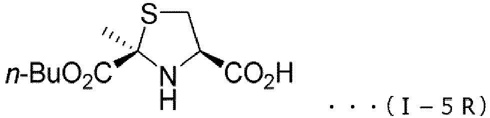

### (Production Example 6)

(2RS,4R)-2-methyl-2,4-thiazolidinedicarboxylic acid 2-t-butyl ester (hereinafter referred to as "Compound 6") was obtained by the following method. The structures of the 2S and 2R compounds of Compound 6 are shown in the following formulas (I-6S) and (I-6R), respectively.

Compound 6 (diastereomeric mixture) was obtained as a white solid using L-cysteine and t-butyl pyruvate as raw materials by the same method as in Production Example 1.

### <Analysis data>

Yield 64 %; 2S compound : ESI MS m/z 247.9 (M+H)⁺; ¹H NMR (400 MHz, CD₃OD) δ 4.32 (1H, dd, J=7.2, 8.0 Hz), 3.37 (1H, dd, J=7.2, 10.4 Hz), 3.13 (1H, dd, J=8.0, 10.4 Hz), 1.80 (3H, s), 1.49 (9H, s); 2R compound : ESI MS m/z 246.0 (M-H)⁻; ¹H NMR (400 MHz, CD₃OD) δ 4.08 (1H, dd, J=6.0, 8.0 Hz), 3.43 (1H, dd, J=6.0, 10.4 Hz), 2.90 (1H, t, J=10.4 Hz), 1.66 (3H, s), 1.52 (9H, s).

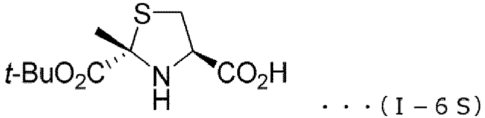

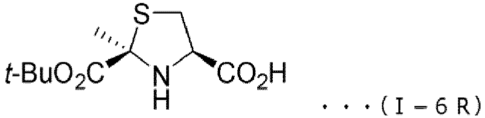

### (Production Example 7)

(2RS,4R)-2-methyl-2,4-thiazolidinedicarboxylic acid 2-n-octyl ester (hereinafter referred to as "Compound 7") was obtained by the following method. The structures of the 2S and 2R compounds of Compound 7 are shown in the following formulas (I-7S) and (I-7R), respectively.

Compound 7 (diastereomeric mixture) was obtained as a white waxy substance using L-cysteine and n-octyl pyruvate as raw materials by the same method as in Production Example 1.

### <Analysis data>

Yield 44 %; 2S compound : ESI MS m/z 304.0 (M+H)⁺; ¹H NMR (400 MHz, CD₃OD) δ 4.35 (1H, dd, J=6.8, 8.0 Hz), 4.15 (2H, t, J=6.8 Hz), 3.38 (1H, dd, J=6.8, 10.4 Hz), 3.14 (1H, dd, J=8.4, 10.4 Hz), 1.84 (3H, s), 1.74-1.64 (2H, m), 1.43-1.32 (10H, m), 0.94-0.91 (3H, m); 2R compound : ESI MS m/z 304.0 (M+H)⁺; ¹H NMR (400 MHz, CD₃OD) δ 4.10 (1H, dd, J=6.0, 10.0 Hz), 4.27-4.17 (2H, m), 3.46 (1H, dd, J=6.0, 10.4 Hz), 2.93 (1H, dd, J=10.0, 10.4 Hz), 1.74-1.64 (5H, m), 1.43-1.32 (10H, m), 0.94-0.91 (3H, m).

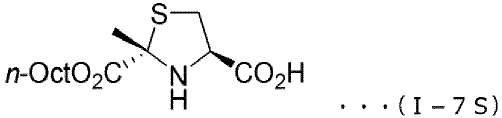

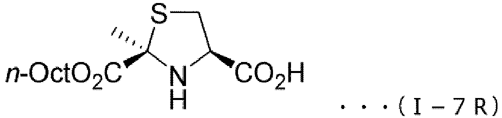

### (Production Example 8)

(2RS,4R)-2,4-thiazolidinedicarboxylic acid 2-ethyl ester (hereinafter referred to as "Compound 8") was obtained by the following method. The structures of the 2S and 2R compounds of Compound 8 are shown in the following formulas (I-8S) and (I-8R), respectively.

Compound 8 (diastereomeric mixture) was obtained as a white solid using L-cysteine and ethyl glyoxylate as raw materials by the same method as in Production Example 1.

### <Analysis data>

Yield 67 %; 2S compound: ESI MS m/z 205.9 (M+H)⁺; ¹H NMR (400 MHz, CD₃OD) δ 4.38 (1H, dd, J=6.0, 6.4 Hz), 4.31-4.24 (2H, m), 3.27 (1H, dd, J=6.4, 10.4 Hz), 3.11 (1H, dd, J=6.0, 10.4 Hz), 1.29 (3H, t, J=7.2 Hz); 2R compound: ESI MS m/z 206.0
(M+H)⁺; ¹H NMR (400 MHz, CD₃OD) δ 4.23-4.19 (2H, m), 3.96 (1H, dd, J=6.4, 10.0 Hz), 3.38 (1H, dd, J=6.4, 10.0 Hz), 2.89 (1H, t, J=10.0 Hz), 1.31 (3H, t, J=6.8 Hz).

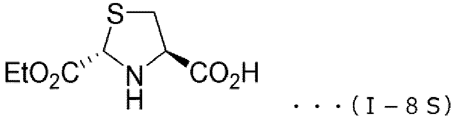

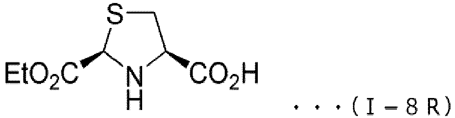

### (Production Example 9)

(2RS,4R)-2-(4-hydroxyphenyl)methyl-2,4-thiazolidinedicarboxylic acid 2-methyl ester (hereinafter referred to as "Compound 9") was obtained by the following method. The structures of the 2S and 2R compounds of Compound 9 are shown in the following formulas (I-9S) and (I-9R), respectively.

Compound 9 (diastereomeric mixture) was obtained as a light brown candy-like substance using L-cysteine and methyl (4-hydroxyphenyl)pyruvate as raw materials by the same method as in Production Example 1. To an aqueous suspension of Compound 9, an equimolar amount of sodium bicarbonate was added and dissolved, and then the solution was concentrated and dried under reduced pressure, to obtain the corresponding Na salt as a light brown solid. The Na salt was used in subsequent experiments.

### <Analysis data>

Yield 61 %; 2S compound : ESI MS m/z 297.9 (M+H)⁺; ¹H NMR (400 MHz, CD₃OD) δ 7.17 (2H, d, J=8.4 Hz), 6.74 (2H, d, J=8.4 Hz), 4.19 (1H, dd, J=6.4, 8.4 Hz), 3.73 (3H, s), 3.40-3.32 (2H, m), 3.26 (1H, dd, J=6.4, 10.0 Hz), 2.84 (1H, dd, J=8.4, 10.0 Hz). 2R compound : ESI MS m/z 297.9 (M+H)⁺; ¹H NMR (400 MHz, CD₃OD) δ 7.13 (2H, d, J=8.4 Hz), 6.68 (2H, d, J=8.4 Hz), 3.89 (1H, dd, J=6.4, 8.4 Hz), 3.74 (3H, s), 3.19-3.08 (2H, m), 2.80 (1H, t, J=10.0 Hz).

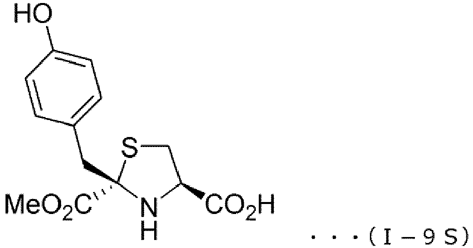

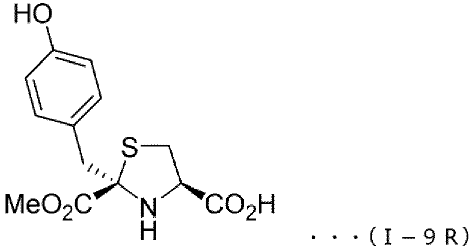

### (Production Example 10)

(2RS,4R)-2-i-propyl-2,4-thiazolidinedicarboxylic acid 2-ethyl ester (hereinafter referred to as "Compound 10") was obtained by the following method. The structures of the 2S and 2R compounds of Compound 10 are shown in the following formulas (I-10S) and (I-10R), respectively.

Compound 10 (diastereomeric mixture, mainly containing the 2S compound) was obtained as a white solid using L-cysteine and ethyl 2-ketoisovalerate as raw materials by the same method as in Production Example 1.

### <Analysis data>

Yield 54 %; 2S compound : ESI MS m/z 248.0 (M+H)⁺; ¹H NMR (400 MHz, CD₃OD) δ 4.26-4.21 (2H, m), 4.13 (1H, dd, J=6.4, 9.2 Hz), 3.29 (1H, dd, J=6.4, 10.4 Hz), 2.85 (1H, t, J=9.6 Hz), 2.48 (1H, m), 1.30 (3H, t, J=7.2 Hz), 1.16 (3H, d, J=7.2 Hz), 1.08 (3H, d, J=6.8 Hz); 2R compound : ESI MS m/z 247.9 (M+H)⁺.

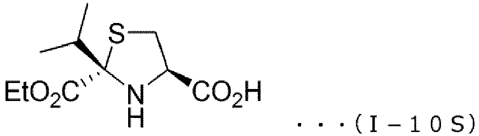

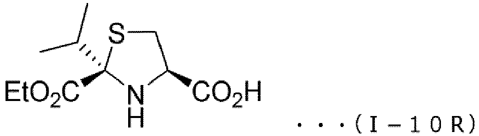

### (Production Example 11)

(2RS,4R)-2-i-butyl-2,4-thiazolidinedicarboxylic acid 2-methyl ester (hereinafter referred to as "Compound 11") was obtained by the following method. The structures of the 2S and 2R compounds of Compound 11 are shown in the following formulas (I-11S) and (I-11R), respectively.

Compound 11 (diastereomeric mixture) was obtained as a colorless candy-like substance using L-cysteine and methyl 2-ketoisocaproate as raw materials by the same method as in Production Example 1. To an aqueous suspension of Compound 11, an equimolar amount of sodium bicarbonate was added and dissolved, and then the solution was concentrated and dried under reduced pressure, to obtain the corresponding Na salt as a white solid. The Na salt was used in subsequent experiments.

### <Analysis data (free compound)>

Yield 58 %; 2S compound: ESI MS m/z 247.9 (M+H)⁺; ¹H NMR (400 MHz, CD₃OD) δ 4.26 (1H, dd, J=6.8, 8.8 Hz), 3.73 (3H, s), 3.29 (1H, dd, J=6.8, 10.4 Hz), 2.98 (1H, dd, J=8.8, 10.4 Hz), 2.30 (1H, dd, J=8.0, 14.4 Hz), 1.99 (1H, dd, J=5.6, 14.4 Hz), 1.83 (1H, m), 1.02 (3H, d, J=6.8 Hz), 0.89 (3H, d, J=6.8 Hz); 2R compound: ESI MS m/z 248.0 (M+H)⁺; ¹H NMR (400 MHz, CD₃OD) δ 4.00 (1H, dd, J=6.0, 10.0 Hz), 3.80 (3H, s), 3.38 (1H, dd, J=6.0, 10.0 Hz), 2.80 (1H, t, J=10.0 Hz), 2.05 (1H, dd, J=6.8, 14.4 Hz), 1.90 (1H, dd, J=6.0, 14.4 Hz), 1.74 (1H, m), 0.98 (3H, d, J=6.4 Hz), 0.84 (3H, d, J=6.8 Hz).

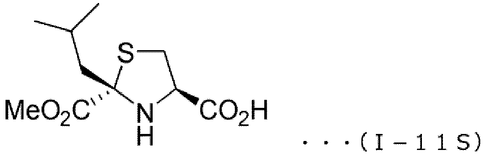

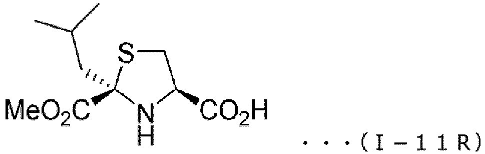

### Example 5: Evaluation of effect of addition of L-cysteine derivatives to feed medium on proliferation of CHO cells and antibody production (2)

### (1) Preparation of basal medium

CELLiST (registered trademark) Basal media (Ajinomoto Co., Ltd.: BASAL3; containing 1.3 mM L-cysteine) was added with LR3-IGF-1 (SIGMA-ALDRICH: I1271-1MG, final concentration of 50 µg/mL) and L-Glutamine (Thermo Fisher SCIENTIFIC: 25030081, final concentration of 6 mM), to prepare a basal medium.

### (2) Preparation of feed medium

Feed media each containing 82 g/L Cellvento (registered trademark) Feed-210 (Merck: 1.02488.0005), 70 g/L glucose (Wako Pure Chemical Industries, Ltd.: 043-31163), and L-cysteine, Compound 3S, or Compound 3R each at 14.5 mM, 29 mM, or 58 mM were prepared. Separately, a tyrosine concentrate (L-tyrosine disodium salt dihydrate (Merck: 122666-87-9) 74.82 g/L, pH 11.3) was prepared.

### (3) Culture experiment

A 3 mL-aliquot of a CHO cell suspension adjusted to 3×10⁵ cells/mL using the prepared basal medium was seeded into a 6 well plate (CORNING, Cat. No. CLS3471), and cultured for 14 days. The culture was carried out with n=2 for all experimental groups. The culture temperature was 37°C and the agitation speed was 110 rpm. A 0.18 mL-aliquot of each of the prepared feed media and a 12 µL-aliquot of the tyrosine concentrate were added on days 4, 7, 9, and 11 from the start of the culture. A cell culture broth was sampled on days 4, 7, 9, 11, and 14 from the start of the culture, and the viable cell count was measured using a viable/dead cells autoanalyzer Vi-CELL^{™} XR (Beckman Coulter), and the antibody production amount on days 11 and 14 from the start of the culture was measured using Octet QK (FORTEBIO).

### (4) Culture results

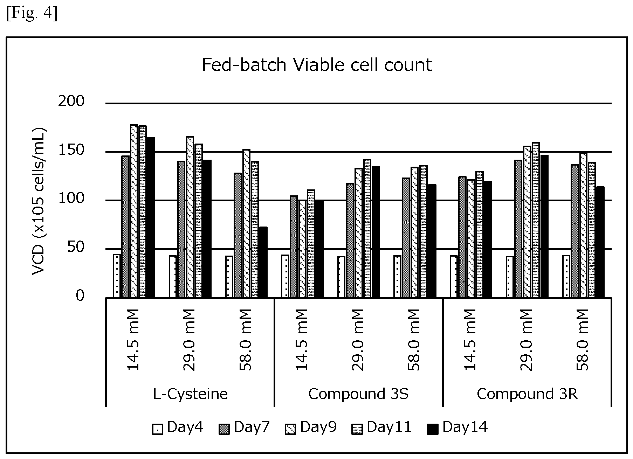

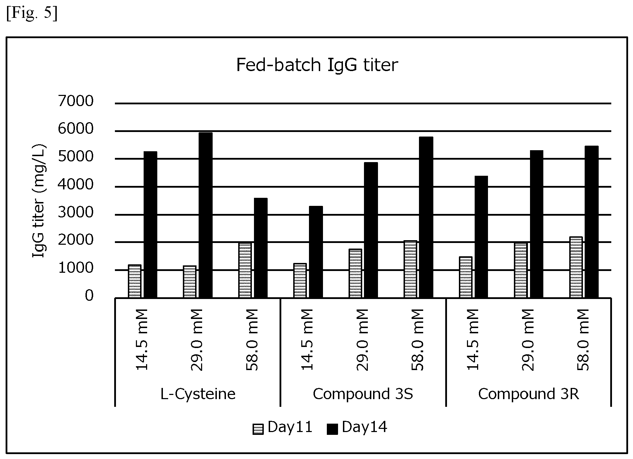

The results are shown in Figs. 4 and 5. When L-cysteine in the feed medium was substituted with Compound 3S or Compound 3R, proliferation of cells was observed at 14.5 mM although proliferation performance of cells was reduced to approximately 70%, and the cell count increased in a concentration-dependent manner. The IgG production amount was also increased in a concentration-dependent manner in both cases of using Compound 3S and Compound 3R, and the IgG production amount of the groups adding 58.0 mM of the compounds was equivalent to that of the group adding 14.5 mM or 29.0 mM of L-cysteine hydrochloride monohydrate. These results indicate that Compound 3 is able to substitute for L-cysteine and that the 2S and 2R compounds of Compound 3 are equivalently effective.

### Example 6: Evaluation of effect of addition of L-cysteine derivatives to feed medium on proliferation of CHO cells and antibody production (3)

### (1) Preparation of basal medium

CELLiST (registered trademark) Basal media (Ajinomoto Co., Ltd.: BASAL3; containing 1.3 mM L-cysteine) was added with LR3-IGF-1 (SIGMA-ALDRICH: I1271-1MG, final concentration of 50 µg/mL) and L-Glutamine (Thermo Fisher SCIENTIFIC: 25030081, final concentration of 6 mM), to prepare a basal medium.

### (2) Preparation of feed medium

Feed media each containing 82 g/L Cellvento (registered trademark) Feed-210 (Merck: 1.02488.0005), 70 g/L glucose (Wako Pure Chemical Industries, Ltd.: 043-31163), and L-cysteine, Compound 4, Compound 8, Compound 9, or Compound 11 each at 58 mM were prepared. In addition, a feed medium without addition of L-cysteine, which only contained 82 g/L Cellvento (registered trademark) Feed-210 and 70 g/L glucose, was prepared. Separately, a tyrosine concentrate (L-tyrosine disodium salt dihydrate (Merck: 122666-87-9) 74.82 g/L, pH 11.3) was prepared.

### (3) Culture experiment

A 30 mL-aliquot of a CHO cell suspension adjusted to 3×10⁵ cells/mL using the prepared basal medium was seeded into 125 mL-volume flasks (CORNING: 431143), and cultured for 11 days. The culture was carried out with n=2 for all experimental groups. The culture temperature was 37°C and the agitation speed was 110 rpm. A 1.8 mL-aliquot of each of the prepared feed media and a 120 µL-aliquot of the tyrosine concentrate were added on days 4, 7, and 9 from the start of the culture. A cell culture broth was sampled on days 4, 7, 9, and 11 from the start of the culture, and the viable cell count was measured using a viable/dead cells autoanalyzer Vi-CELL^{™} XR (Beckman Coulter), and the antibody production amount was measured using Octet

### QK (FORTEBIO).

### (4) Culture results

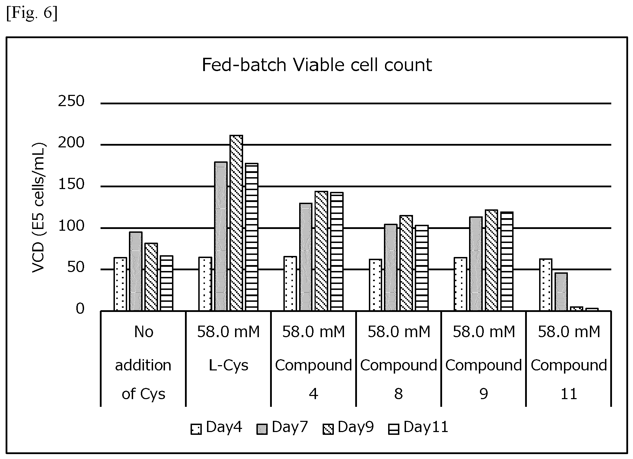

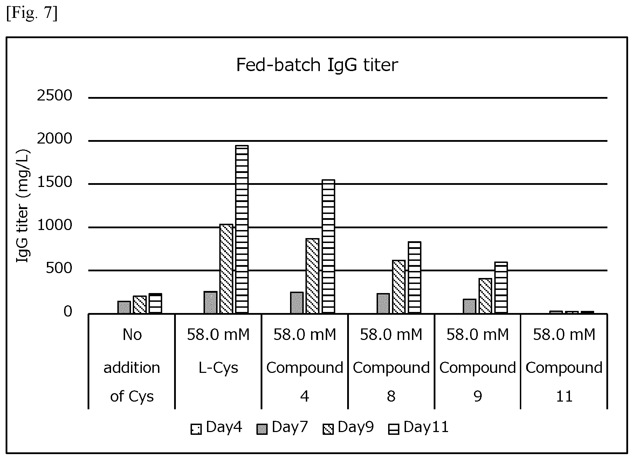

The results are shown in Figs. 6 and 7. The cell count and the antibody production amount observed for the experimental group using Compound 4, Compound 8, or Compound 9 were higher than those of the case without addition of L-cysteine, which indicates that these compounds are able to substitute for L-cysteine. By contrast, the cell count and the antibody production amount decreased in the experimental group using Compound 11 as compared to the case without addition of L-cysteine.

### Example 7: Evaluation of stability of L-cysteine derivatives in buffer solution

In this Example, the stability of L-cysteine derivatives in a buffer solution was evaluated. As the L-cysteine derivatives, Compounds 1, 2, 3S, 3R, 4, 6, 8, and 9 were used.

PBS Tablets (Takara Bio, product code: T900) were dissolved in distilled water, to obtain PBS buffer of 9.57 mM, pH 7.4. L-cysteine or Compound 1, 2, 3S, 3R, 4, 6, 8, or 9 was dissolved in PBS buffer at 58 mM, and sodium bicarbonate water was added thereto as necessary to attain the pH of 7.0 to 7.2, to obtain 9 solutions in total. All of these solutions were prepared as transparent solutions.

After each of the solutions was stored at 5°C in the dark, crystal precipitation was confirmed by visual observation. The results are shown in Table 2. In the table, "-" indicates that no crystal precipitation was observed. In the table, "+" to "++++" indicate that crystal precipitation was observed, and the higher the number of "+", the greater the degree of precipitation. Under the condition of adding L-cysteine, significant precipitation was observed from 3 days after the start of storage. By contrast, under the other conditions, the solutions remained clear until 24 days after the start of storage, and no crystal precipitation was observed. Hence, it was revealed that all of Compounds 1, 2, 3S, 3R, 4, 6, 8, and 9 are more stable than L-cysteine in the culture medium.

**Table 2. Stability of compounds in buffer solution**

| Compounds | Buffer pH | Days | | | | | |
|---|---|---|---|---|---|---|---|
| | | 0 | 3 | 7 | 14 | 21 | 24 |
| 58 mM L-Cysteine | 7.0 to 7.2 | - | ++ | +++ | +++ | +++ | +++ |
| 58 mM Compound 1 | | - | - | - | - | - | - |
| 58 mM Compound 2 | | - | - | - | - | - | - |
| 58 mM Compound 3S | | - | - | - | - | - | - |
| 58 mM Compound 3R | | - | - | - | - | - | - |
| 58 mM Compound 4 | | - | - | - | - | - | - |
| 58 mM Compound 6 | | - | - | - | - | - | - |
| 58 mM Compound 8 | | - | - | - | - | - | - |
| 58 mM Compound 9 | | - | - | - | - | - | - |

### Industrial Applicability

According to the present invention, animal cells can be cultured. In an embodiment, according to the present invention, an objective substance such as a protein can be produced by using animal cells.

## Claims

1. A culture medium for culturing animal cells, the culture medium containing an active ingredient,
wherein the active ingredient is a compound shown by the following formula (I): 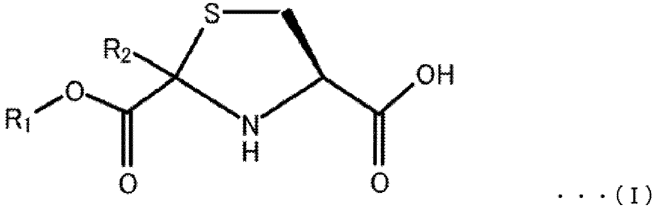
wherein, in the formula (I), "Ri" represents a C1-22 alkyl group that may have a substituent, and "R₂" represents a hydrogen atom, a C6-12 aryl group that may have a substituent, or a linear C1-6 alkyl group that may have a substituent.

2. The culture medium according to claim 1, wherein:
"Ri" represents a C1-12 alkyl group, or a C1-6 alkyl group substituted with a C6-12 aryl group that may have a substituent, and/or
"R₂" represents a hydrogen atom, a linear C1-6 alkyl group, a linear C1-6 alkyl group substituted with a C6-12 aryl group that may have a substituent, or a linear C1-6 alkyl group substituted with a C1-7 acyl group.

3. The culture medium according to claim 1 or 2, wherein the active ingredient is a compound selected from the group consisting of (2RS,4R)-2-methyl-2,4-thiazolidinedicarboxylic acid 2-ethyl ester, (2RS,4R)-2-methyl-2,4-thiazolidinedicarboxylic acid 2-methyl ester, (2RS,4R)-2-methyl-2,4-thiazolidinedicarboxylic acid 2-i-propyl ester, (2RS,4R)-2-methyl-2,4-thiazolidinedicarboxylic acid 2-n-butyl ester, (2RS,4R)-2-methyl-2,4-thiazolidinedicarboxylic acid 2-t-butyl ester, (2RS,4R)-2-methyl-2,4-thiazolidinedicarboxylic acid 2-n-octyl ester, (2RS,4R)-2,4-thiazolidinedicarboxylic acid 2-ethyl ester, and (2RS,4R)-2-(4-hydroxyphenyl)methyl-2,4-thiazolidinedicarboxylic acid 2-methyl ester, and the 2R and 2S compounds thereof.

4. The culture medium according to any one of claims 1 to 3, wherein the active ingredient is a compound selected from the group consisting of (2RS,4R)-2-methyl-2,4-thiazolidinedicarboxylic acid 2-ethyl ester and the 2R and 2S compounds thereof.

5. The culture medium according to any one of claims 1 to 3, wherein the active ingredient is a compound selected from the group consisting of (2RS,4R)-2-methyl-2,4-thiazolidinedicarboxylic acid 2-methyl ester and the 2R and 2S compounds thereof.

6. The culture medium according to any one of claims 1 to 5, wherein the concentration of the active ingredient is 0.92 mM or more.

7. The culture medium according to any one of claims 1 to 6, wherein the concentration of the active ingredient is 5 mM or more.

8. The culture medium according to any one of claims 1 to 7, wherein the concentration of the active ingredient is 10 mM or more.

9. The culture medium according to any one of claims 1 to 8, wherein the culture medium is a feed medium.

10. The culture medium according to any one of claims 1 to 8, wherein the culture medium is a starting medium.

11. A method for producing an objective substance, the method comprising:
culturing animal cells having an objective substance-producing ability in a culture medium; and
collecting the objective substance,
wherein the culturing is carried out in the presence of an active ingredient, and
wherein the active ingredient is a compound shown by the following formula (I): 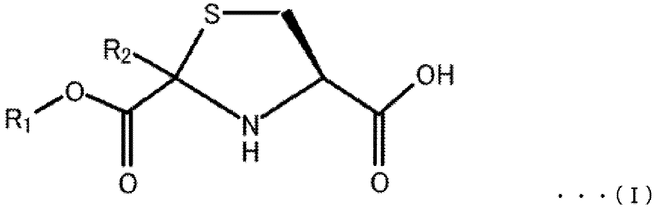
wherein, in the formula (I), "Ri" represents a C1-22 alkyl group that may have a substituent, and "R₂" represents a hydrogen atom, a C6-12 aryl group that may have a substituent, or a linear C1-6 alkyl group that may have a substituent.

12. The method according to claim 11, wherein the objective substance is a protein.

13. A method for culturing animal cells, the method comprising:
culturing the animal cells in a culture medium,
wherein the culturing is carried out in the presence of an active ingredient, and
wherein the active ingredient is a compound shown by the following formula (I): 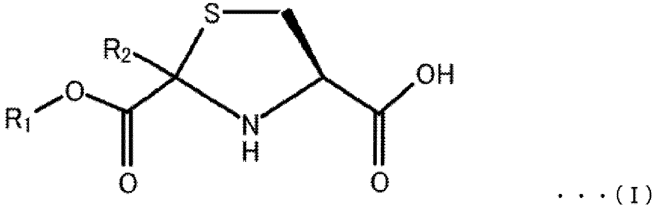
wherein, in the formula (I), "Ri" represents a C1-22 alkyl group that may have a substituent, and "R₂" represents a hydrogen atom, a C6-12 aryl group that may have a substituent, or a linear C1-6 alkyl group that may have a substituent.

14. The method according to any one of claims 11 to 13, wherein:
"Ri" represents a C1-12 alkyl group, or a C1-6 alkyl group substituted with a C6-12 aryl group that may have a substituent, and/or
"R₂" represents a hydrogen atom, a linear C1-6 alkyl group, a linear C1-6 alkyl group substituted with a C6-12 aryl group that may have a substituent, or a linear C1-6 alkyl group substituted with a C1-7 acyl group.

15. The method according to any one of claims 11 to 14, wherein the active ingredient is a compound selected from the group consisting of (2RS,4R)-2-methyl-2,4-thiazolidinedicarboxylic acid 2-ethyl ester, (2RS,4R)-2-methyl-2,4-thiazolidinedicarboxylic acid 2-methyl ester, (2RS,4R)-2-methyl-2,4-thiazolidinedicarboxylic acid 2-i-propyl ester, (2RS,4R)-2-methyl-2,4-thiazolidinedicarboxylic acid 2-n-butyl ester, (2RS,4R)-2-methyl-2,4-thiazolidinedicarboxylic acid 2-t-butyl ester, (2RS,4R)-2-methyl-2,4-thiazolidinedicarboxylic acid 2-n-octyl ester, (2RS,4R)-2,4-thiazolidinedicarboxylic acid 2-ethyl ester, and (2RS,4R)-2-(4-hydroxyphenyl)methyl-2,4-thiazolidinedicarboxylic acid 2-methyl ester, and the 2R and 2S compounds thereof.

16. The method according to any one of claims 11 to 15, wherein the active ingredient is a compound selected from the group consisting of (2RS,4R)-2-methyl-2,4-thiazolidinedicarboxylic acid 2-ethyl ester and the 2R and 2S compounds thereof.

17. The method according to any one of claims 11 to 15, wherein the active ingredient is a compound selected from the group consisting of (2RS,4R)-2-methyl-2,4-thiazolidinedicarboxylic acid 2-methyl ester and the 2R and 2S compounds thereof.

18. The method according to any one of claims 11 to 17, wherein the active ingredient is supplied to the culture medium after the start of the culturing.

19. The method according to any one of claims 11 to 18, wherein the active ingredient is contained in the culture medium at the start of the culturing.

20. The method according to any one of claims 11 to 19, wherein the concentration of the active ingredient in the culture medium during the culturing is 0.92 mM or more.

21. The method according to any one of claims 11 to 20, wherein the concentration of the active ingredient in the culture medium during the culturing is 1.5 mM or more.

22. The method according to claim 20 or 21, wherein the active ingredient is supplied to the culture medium after the start of the culturing so as to be contained in the culture medium at said concentration.

23. The method according to any one of claims 20 to 22, wherein the active ingredient is contained in the culture medium at said concentration at the start of the culturing.

24. The method according to any one of claims 20 to 23,
wherein the active ingredient is contained in the culture medium at said concentration in terms of the average value over a specific period of the culturing, and
wherein the specific period is the whole period of the culturing or a period where the viable cell density of the animal cells in the culture medium is 5×10⁶ cells/mL or more.

25. The method according to any one of claims 11 to 24,
wherein the active ingredient is supplied to the culture medium at a supply amount of 0.1 mM or more per day over a specific period of the culturing, and
wherein the specific period is the whole period of the culturing or a period where the viable cell density of the animal cells in the culture medium is 5×10⁶ cells/mL or more.

26. The method according to any one of claims 11 to 25,
wherein the active ingredient is supplied to the culture medium at a supply amount of 0.3 mM or more per day over a specific period of the culturing, and
wherein the specific period is the whole period of the culturing or a period where the viable cell density of the animal cells in the culture medium is 5×10⁶ cells/mL or more.

27. The method according to any one of claims 11 to 26, wherein the culturing comprises carrying out perfusion culture using a feed medium containing the active ingredient.

28. The method according to any one of claims 11 to 27,
wherein the culturing is carried out in the presence of a cysteine-related substance, and
wherein the cysteine-related substance is selected from cysteine and derivatives thereof other than the active ingredient.

29. The method according to claim 28, wherein the cysteine-related substance is selected from cysteine, cystine, and cysteinyl pyruvic acid.

30. The method according to claim 28 or 29, wherein the cysteine-related substance is contained in the culture medium at the start of the culturing.

31. A method for producing a compound shown by the following formula (I), the method comprising:
reacting L-cysteine and a pyruvate derivative shown by the following formula (II) in a hydrous medium to thereby form crystals of the compound; and
collecting the crystals: 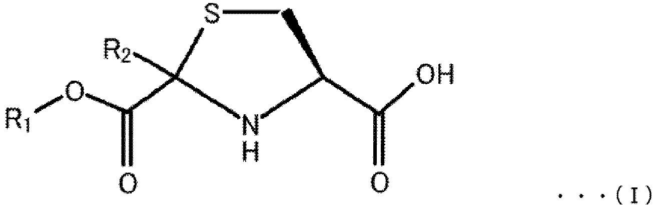 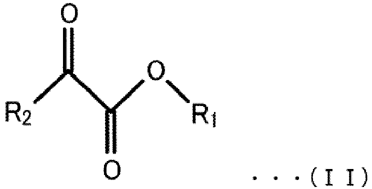
wherein, in the formulas (I) and (II), "Ri" represents a C1-C6 alkyl group, and "R₂" represents methyl group.
